# EUROPÄISCHE PATENTSCHRIFT

(11) **EP 2 160 383 B1**
(45) Veröffentlichungstag und Bekanntmachung des Hinweises auf die Patenterteilung: **09.03.2016**
(21) Anmeldenummer: 08760224.9
(22) Anmeldetag: 29.05.2008
(51) Int. Cl.: C07D 251/70

(54) **N, N',N''-TRIMETHYL-N, N', N''-TRIS(METHOXYCARBONYL)-MELAMINDERIVATE UND ÄHNLICHE TRIAZINDERIVATE ALS VERNETZER UND/ODER BINDEMITTEL IN BESCHICHTUNGSMASSEN, LACKEN, FARBEN, VERBUNDWERKSTOFFEN UND LAMINATEN**
N, N',N''-TRIMETHYL-N, N', N''-TRIS(METHOXYCARBONYL)-MELAMINE DERIVATIVES AND SIMILAR TRIAZINE DERIVATIVES FOR AS CROSS-LINKERS AND/OR BINDERS IN COATING COMPOUNDS, PAINTS, INKS, COMPOSITE MATERIALS AND LAMINATES
DÉRIVÉS DE N, N',N''-TRIMÉTHYL-N, N', N''-TRIS(MÉTHOXYCARBONYL)-MÉLAMINE ET DÉRIVÉS DE TRIAZINE ANALOGUES EN TANT QU'AGENTS DE RÉTICULATION ET/OU LIANTS DANS DES PRODUITS DE REVÊTEMENT, DES VERNIS, DES PEINTURES, DES MATÉRIAUX COMPOSITES ET DES STRATIFIÉS

(30) Priorität: 31.05.2007 DE 102007025451
(43) Veröffentlichungstag der Anmeldung: 10.03.2010
(73) Patentinhaber: Borealis Agrolinz Melamine GmbH, 4021 Linz (AT)
(72) Erfinder: DICKE, René, A-4050 Leonding (AT); BURGER, Martin, D-84032 Altdorf (DE); HAHN, Christoph, A-4040 Linz (AT); ENDESFELDER, Andreas, A-4021 Linz (AT); SCHWARZINGER, Clemens, 4600 Wels (AT); BRETTERBAUER, Klaus, 4020 Linz (AT); SCHMIDT, Harald, 4040 Linz (AT)
(74) Vertreter: Maikowski & Ninnemann
(86) Internationale Anmeldenummer: PCT/EP2008/056633
(87) Internationale Veröffentlichungsnummer: WO 2008/145704

(56) Entgegenhaltungen:
- EP-A- 0 226 536
- WO-A-2004/054990
- WO-A-2005/030735
- WO-A-2005/100326
- WO-A-2005/100328
- WO-A-2005/103016
- US-A- 2 394 042
- US-A- 4 007 032
- US-A- 5 084 541
- US-A- 5 705 641
- US-A- 6 063 922
- US-B1- 6 204 381
- DATABASE BEILSTEIN BEILSTEIN INSTITUTE FOR ORGANIC CHEMISTRY, FRANKFURT-MAIN, DE; 1984, XP002498659 Database accession no. BRN: 93538 & LIO ET AL: BULLETIN OF THE CHEMICAL SOCIETY OF JAPAN, Bd. 57, Nr. 4, 1984, Seiten 2009-2010,

## Beschreibung

Die Erfindung betrifft Triazinderivate, insbesondere modifizierte Triazincarbamate und Triazinharnstoffe, ein Verfahren zu deren Herstellung sowie deren Verwendung.

Die US 5,084,541 beschreibt Tricarbamoyltriazine (Triazintricarbamate, Melamintricarbamate), die ausgehend von Triazintriisocyanurat durch Umsetzung mit verschiedenen Alkoholen synthetisiert werden. Diese stets dreifach substituierten Produkte können beispielsweise als Vernetzer eingesetzt werden.

Die US 6,063,922 beschreibt Triazincarbamate, die aus einer Umsetzung mit Melamin und acyclischen organischen Carbonaten in Gegenwart von starken Basen gebildet werden. Dabei werden stets Bi- oder Tricarbamate gebildet.

Die EP 0 366 884 A2 beschreibt Triazinverbindungen mit mindestens einer Carbamylgruppe, wobei die Carbamylgruppe durch zumindest eine Methylengruppe von der Aminogruppe eines Triaminotriazins getrennt ist. Zu den Verbindungen der EP 0 366 884 A2 zählen auch Produkte aus Melamin und Formaldehyd.

Die DE 10 2004 018 543 A1 beschreibt ebenfalls carbamatgruppenhaltige Triazinderivate, die als Vernetzer für Lacke mit verbesserten Eigenschaften dienen.

Die WO 2005/103016 A1 beschreibt strahlungshärtende Triazincarbamate und Triazinharnstoffe, die halogenfrei sind und Vinyl-, Methacryloyl- oder Acryloylgruppen enthalten.

WO 2005/100326 A1 betrifft spezifische Carbamat-funktionalisierte Triazinderivate, die als Crosslinker für filmbildende Bindemittel einsetzbar sind.

WO 2005/100328 A1 betrifft Ureido- und Carbamat-Triazine und Verfahren zu deren Herstellung, wobei die Triazinderivate über bis zu 3 Ureido- oder Carbamatgruppen verfügen können. Die N-Atome der Ureido- bzw. Carbamatgruppen sind jedoch nicht substituiert und verfügen somit jeweils über ein unsubstituiertes Wasserstoff-Atom. Die Aminogruppen der Triazinderivate können hier entweder in Form einer primären Aminogruppe oder in Form von sekundären Aminogruppen wie z.B. in Form der sekundären Ureido- bzw. Carbamatgruppen vorliegen.

US 6,204,381 beschreibt mit Verfahren zur Herstellung von 1,3,5-Triazincarbamaten durch Umsetzung von 1,3,5-Triazinderivaten mit geeigneten Abgangsgruppen mit Cyanat-Gruppen enthaltenen Substraten und einer Isocyanat- reaktiven Verbindung in einem geeigneten Lösungsmittel. Die mittels dieses Verfahrens hergestellten Triazincarbamate können neben 2 Carbamatgruppen als weitere Substituenten unter anderem verschiedene Kohlenwasserstoffreste aufweisen.

WO 2004/054990 A2 offenbart ein Verfahren zur Herstellung von Triazincarbamaten durch Umsetzung eines Triazins mit einem zyklischen Kohlensäureester in Gegenwart eines Alkohols oder eines Alkali- oder Erdalkalialkanolates als Base. Die hergestellten Triazincarbamate können mindestens eine Carbamatgruppe und als weitere Substituenten verschiedene Alkylreste oder mit Alkylresten substituierte Aminogruppen aufweisen. Darüber hinaus kann das an den Triazinring gebundene N-Atom der Carbamatgruppe ebenfalls mit einem Alkylrest substituiert sein.

EP 226536 A2 offenbart Triazinderivate mit mindestens einer Carbamatgruppe und Aminogruppen, wobei die Aminogruppen mit verschiedenen Alkylresten (R¹, R²) substituiert vorliegen können. Es ist jedoch auch möglich, dass eine der Aminogruppen unsubstituiert (R²=H) vorliegt, so dass lediglich eine Aminogruppen mit einem Alkylrest substituiert (R¹) ist.

US 4,007,032 betrifft Triazincarbamate mit mindestens einer Carbamatgruppe, wobei das an den Triazin-Ring gebundene N-Atom der Carbamatgruppe mit einem Alkylrest (R³) substituiert vorliegen kann. Der Triazinring kann als weitere Substituenten eine mit Alkylresten substituierte Aminogruppen und eine Methoxy-, Ethoxy-, Methlythio-, Ethylthio- oder Azido-Gruppe (R⁵) aufweisen.

WO 2005/030735 A1 betrifft Triazinderivate mit einer Ureidogruppe, wobei das an den Triazinring gebundene N-Atom mit verschiedenen Kohlenwasserstoffresten (R¹) substituiert sein kann. Der Triazinring kann des Weiteren mit verschiedensten Resten R³ und R⁴ substituiert sein.

US 2,394, 042 offenbart Triazinderivate mit einer Ureidogruppe, wobei das an den Triazinring gebundene N-Atom durch einen Alkylrest substituiert sein kann. Weitere an den Triazinring gebundene Substituenten können mit Alkylresten substituierte Aminogruppen darstellen.

Der Erfindung liegt das Problem zugrunde, Triazinderivate bereitzustellen, die sich gegenüber den bekannten Verbindungen in ihrer Viskosität bzw. ihren Schmelzeigenschaften einstellen lassen.

Dieses Problem wird durch Triazinderivate gemäß der Formel (I) entsprechend der Ansprüche 1 und 2 gelöst. Bei den erfindungsgemäßen Triazinderivaten handelt es sich insbesondere um modifizierte Di- und Tri-Ureido-Triazine, Melamincarbamate und Melaminharnstoffe sowie Guanamincarbamate und Guanaminharnstoffe.

Die Triazinderivate gemäß Anspruch 1 weisen eine Struktur der allgemeinen Formel (Ia) auf 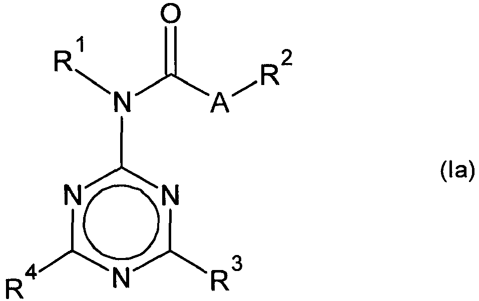 , wobei
- **R³** Q¹ oder einen mit seinem zentralen Stickstoffatom an den Triazinring der Struktur der Formel (la) gebundenen Rest der Formel **R⁵-N-R⁶** bedeutet, wobei
   - Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
- **R⁴** einen mit dem Stickstoffatom an den Triazinring der Struktur der Formel (la) gebundenen Rest der Formel **R⁷-N-R⁸** bedeutet,
- **R¹, R⁵,** und **R⁷** unabhängig voneinander Q² bedeuten, wobei
   - Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
- **A** -NH-, **-NR²-, -NR⁶²-** oder **-NR⁸²-,** insbesondere jedoch nur **-NR²-,** bedeutet,
- **R⁶** Q², -CO**-A-R²,** -CO**-A-R⁶¹,** insbesondere jedoch nur -CO**-A-R²,** bedeutet,
- R⁸ -CO-A-R², -CO-A-R⁸¹ bedeutet,
- **R², R⁶¹, R⁶², R⁸¹** und **R⁸²** unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) 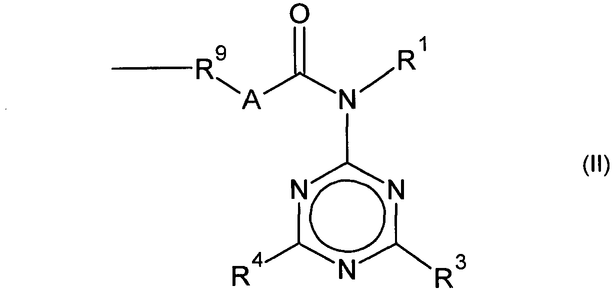 bedeuten,
   - wobei **R⁹** Q² bedeutet,
- mit der Maßgabe, dass, sofern **R³ R⁵-**N**-R⁶** und **R⁴ R⁷-**N**-R⁸** bedeuten,
   - mindestens einer der Reste **R¹, R⁵,** und **R⁷** Q² bedeutet und an ein Stickstoffatom gebunden ist, das kovalent mit dem Rest -CO**-A-R²,** -CO**-A-R⁶¹** oder -CO-**A-R⁸¹**
   - verbunden ist (das heißt, dieses Stickstoffatom ist insbesondere Teil einer Carbamatgruppe), oder
   - zumindest die Reste **R⁵** und **R⁶** einerseits oder die Reste **R⁷** und **R⁸** andererseits jeweils Q² bedeuten (also mindestens zwei Reste des Typs Q² an ein und dasselbe Stickstoffatom gebunden sind). Die Triazinderivate gemäß Anspruch 2 weisen eine Struktur der allgemeinen Formel (Ib) 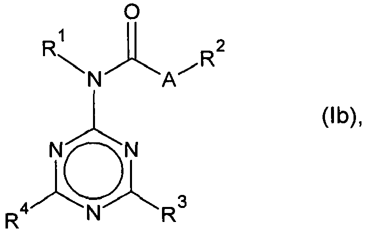 wobei
- **R³** Q¹ oder einen mit dem Stickstoffatom an den Triazinring der Struktur der Formel (Ib) gebundenen Rest der Formel **R⁵-**N**-R⁶** bedeutet, wobei
   - Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
- **R⁴** Q¹ oder einen mit dem Stickstoffatom an den Triazinring der Struktur der Formel (Ib) gebundenen Rest der Formel **R⁷-**N**-R⁸** bedeutet,
- **R¹, R⁵,** und **R⁷** unabhängig voneinander Q² bedeuten, wobei
   - Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
- **A** -O- bedeutet,
- **R⁶** und **R⁸** unabhängig voneinander Q² -CO**-A-R²,** -CO**-A-R⁶¹,** -CO**-A-R⁸¹** bedeuten,
- **R², R⁶¹, R⁶², R⁸¹** und **R⁸²** unabhängig voneinander einen Rest eines substituierten oder nicht substituierten Alkohols ausgewählt aus den Verbindungen 2-Hydroxyethylacrylat, 2-Hydroxypropylacrylat, 4-Hydroxybutylacrylat, 6-Hydroxyhexylacrylat, Diethylenglykolmonoacrylat, Triethylenglykolmonoacrylat, Dipropylenglykolmonoacrylat, Tripropylenglykolmonoacrylat, Trimethylolpropandiacrylat, Pentaerythritdiacrylat, Pentaerythrittriacrylat, 2-Hydroxyethylmethacrylat, 2-Hydroxypropylmethacrylat, 4-Hydroxybutylmethacrylat, 6-Hydroxyhexylmethacrylat, Diethylenglykolmonomethacrylat, Triethylenglykolmonomethacrylat, Dipropylenglykol-monomethacrylat, Tripropylenglykolmonomethacrylat, Trimethylolpropandimethacrylat, Pentaerythritdimethacrylat, Pentaerythrittrimethacrylat und/oder 4-Hydroxybutylvinylether, oder einen Rest der allgemeinen Formel (II) 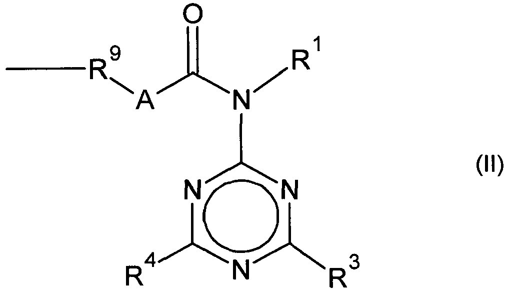 bedeuten,
   - wobei **R⁹** Q² bedeutet,
- mit der Maßgabe, dass, sofern **R³ R⁵-**N**-R⁶** und **R⁴ R⁷-**N**-R⁸** bedeuten,
   - mindestens einer der Reste **R¹, R⁵,** und **R⁷** Q¹ bedeutet und an ein Stickstoffatom gebunden ist, das kovalent mit dem Rest -CO**-A-R²,** -CO**-A-R⁶¹** oder -CO**-A-R⁸¹** verbunden ist, oder
   - zumindest die Reste **R⁵** und **R⁶** einerseits oder die Reste **R⁷** und **R⁸** andererseits jeweils Q² bedeuten.

Das der Erfindung zugrunde liegende Problem wird gleichermaßen durch Mischungen der allgemeinen Formel (I) entsprechender Triazinderivate gelöst.

Als ein durch eine beliebige Gruppe unterbrochener linearer oder verzweigter Kohlenwasserstoffrest (wie insbesondere C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl) wird dabei im Sinne dieser Erfindung nur ein solcher Kohlenwasserstoffrest verstanden, der nicht an einem seiner Enden die entsprechende Gruppe trägt. Vielmehr muss die Kohlenwasserstoffkette tatsächlich von der Gruppe unterbrochen sein.

Das bedeutet, dass der Rest Q¹, wenn er ein lineares oder verzweigtes C₁-C₅₀-Alkyl darstellt, unmittelbar mit einem (unsubstituierten) Kohlenstoffatom an den Triazinring gebunden ist. Da cyclische Substituenten keine Enden im herkömmlichen Sinn aufweisen, können diese cyclischen Substituenten auch mit einem Sauerstoffatom, Schwefelatom, Stickstoffatom oder durch eine Gruppe des Typs -C(O)O-, -OC(O)-, -C(O)-, -NHC(O)O-, -OC(O)NH- oder - OC(O)O- an den Triazinring gebunden sein.

Eine Besonderheit der erfindungsgemäßen Triazinderivate ist darin zu sehen, dass bei Vorhandensein dreier an den Triazinring gebundener, ggf. derivatisierter Aminogruppen, das heißt, wenn dem Rest R³ die Bedeutung R⁵-N-R⁶ und dem Rest R⁴ die Bedeutung R⁷-N-R⁸ zukommt, mindestens eines der insgesamt fünf möglichen Wasserstoffatome an den drei Aminogruppen durch eine Kohlenwasserstoffeinheit des Typs Q² substituiert ist, wobei der eingeführte Substituent Q² an ein Stickstoffatom gebunden ist, das kovalent mit dem Rest -CO-A-R², -CO-A-R⁶¹ oder -CO-A-R⁸¹ verbunden ist, das also benachbart zu einer Carbonylgruppe angeordnet ist.

Eine weitere Besonderheit der erfindungsgemäßen Triazinderivate ist darin zu sehen, dass statt einer bzw. zwei ggf. substituierter Aminogruppen als Reste R³ und R⁴ auch ein oder zwei C₁-C₅₀-Alkylgruppen bzw. cyclische Substituenten (oder eine Mischungen beider Substituentenarten), welche ebenfalls eine Kohlenwasserstoffeinheit enthalten, an den Triazinring gebunden sein können, so dass auch auf diese Weise ein gegenüber einem Wasserstoffatom als Teil einer ggf. einfach substituierten Aminogruppen erhöhter Kohlenwasserstoffanteil in das erfindungsgemäße Triazinderivat eingebracht werden kann. Auf diese Weise lassen sich auch beispielsweise Benzoguanamin- oder Acetoguanaminderivate bereitstellen.

Eine weitere Besonderheit der erfindungsgemäßen Triazinderivate ist auch darin zu sehen, dass, wenn dem Rest R³ die Bedeutung R⁵-N-R⁶ und dem Rest R⁴ die Bedeutung R⁷-N-R⁸ zukommt, entweder R⁵ und R⁶ jeweils Q² bedeuten oder R⁷ und R⁸ jeweils Q² bedeuten. Auch auf diese Weise lässt sich der Kohlenwasserstoffanteil in den Triazinderivaten erhöhen.

Eine weitere Besonderheit der erfindungsgemäßen Triazinderivate ist auch darin zu sehen, dass, wenn dem Rest R³ die Bedeutung R⁵-N-R⁶ und dem Rest R⁴ die Bedeutung R⁷-N-R⁸ zukommt, zumindest R⁵ oder R⁶ Q² bedeutet und R⁷ und R⁸ gleichzeitig beide H bedeuten. Das heißt, eine direkt an den Triazinring gebundene Aminogruppe liegt unsubstituiert vor, während eine zweite direkt an den Triazinring gebundene Aminogruppen zumindest einfach substituiert sein muss. Dies ist eine weitere Möglichkeit, den Kohlenwasserstoffanteil in den Triazinderivaten zu erhöhen.

Mittels der Substitution von Wasserstoffatomen der Triazinderivate durch Kohlenstoffeinheiten wird das Wasserstoffbrückensystem der Triazinderivate gestört. Auf diese Weise lassen sich vorzugsweise - je nach ausgewähltem Substituenten - die Schmelzeigenschaften und die Viskositäten der erfindungsgemäßen Triazinderivate einstellen. Bei einem niedrigeren Gehalt an Wasserstoffbrücken zeigen die Triazinderivate niedrigere Schmelzpunkte bzw. Viskositäten. Auch lassen sich durch einen sehr niedrigen Gehalt an Wasserstoffbrücken flüssige Verbindungen erhalten.

Durch die gezielte Substitution einzelner Wasserstoffatome der Triazinverbindungen durch organische Reste verbessert sich auch die Haftung der erfindungsgemäßen Triazinderivate auf verschiedenen Substraten, wie beispielsweise auf Holz, Kunststoff, Metall, Papier oder Textilien.

Je nach Länge, Art und Anzahl der als (cyclischen) Substituenten verwendeten Kohlenstoffeinheiten kann bevorzugt auch die Hydrophilie und Hydrophobie der Triazinderivate variiert werden, um eine verbesserte Mischbarkeit der erfindungsgemäßen Triazinderivate mit Stoffen anderer Verbindungsklassen bis hin zu einer Löslichkeit in solchen Stoffen anderer Verbindungsklassen zu erreichen. Das heißt, die Kompatibilität der Triazinderivate zu anderen Stoffen lässt sich durch die Wahl des oder der Substituenten in gewünschter Weise beeinflussen. Beispielsweise führt ein Substituent in Form einer C₆-C₁₂-Alkylkette zu einer verbesserten Mischbarkeit des entsprechenden Triazinderivats mit Alkydharzen. Ferner zeigen mit Ethylenglykol-Einheiten verlängerte Aminogruppen beispielsweise eine leichtere Vermischbarkeit mit Polyetherpolyolen.

Dabei spielen für die Hydrophobie des Substituenten insbesondere die Länge der eingesetzten Kohlenstoffketten eine wichtige Rolle und für die Hydrophilie des Substituenten insbesondere die Art und Anzahl der die Kohlenstoffkette unterbrechenden Atome oder Gruppen polaren Charakters eine wichtige Rolle. Die jeweiligen resultierenden van-der-Waals- und hydrophoben Wechselwirkungen sowie die Anzahl der gebildeten Wasserstoffbrücken beeinflussen die Viskosität und das Schmelzverhalten der Triazinderivate maßgeblich. Wenngleich generell eine Erniedrigung der Viskosität bzw. des Schmelzpunktes vorteilhaft ist, ist durch das Einführen von Kohlenwasserstoffresten, die zahlreiche polare Gruppen, wie beispielsweise -C(O)O-, -OC(O)-, -C(O)-, -NHC(O)O-, - OC(O)NH- und/oder -OC(O)O-, aufweisen und folglich eine verstärkte Wasserstoffbrückenbildung erlauben, eine Erhöhung der Viskosität bzw. des Schmelzpunkts gegenüber den aus dem Stand der Technik bekannten Verbindungen denkbar.

Die Reste R⁶¹, R⁶², R⁸¹ und R⁸² können in einer Variante jeweils dem Rest R² entsprechen.

Vorzugsweise bedeutet mindestens einer der Reste R¹, R⁵, R⁶, R⁷ und R⁸, unabhängig von der Bedeutung der Reste R³ und R⁴, Q², wobei auch zwei, drei, vier oder allen Resten die Bedeutung Q² zukommen kann.

In einer bevorzugten Ausgestaltung der Erfindung bedeutet mindestens einer der Reste R¹, R⁵, und R⁷, unabhängig von der Bedeutung der Reste R³ und R⁴, Q², wobei der mindestens eine Rest, dem die Bedeutung Q² zukommt, gleichzeitig an ein Stickstoffatom gebunden ist, das kovalent mit dem Rest -CO-A-R², -CO-A-R⁶¹ oder -CO-A-R⁸¹ verbunden ist. Diese Bedeutung und chemische Umgebung können auch zwei oder drei der Reste R¹, R⁵ und R⁷ aufweisen.

Bevorzugt bedeutet mindestens einer der Reste R⁵, R⁶, R⁷ und R⁸, unabhängig von der Bedeutung der Reste R³ und R⁴, Q². Diese ausschließliche Bedeutung von Q² für R⁵, R⁶, R⁷ oder R⁸ können auch zwei, drei oder alle dieser Reste tragen

Vorzugsweise weist der cyclische Substituent, den die Reste R³ und R⁴ darstellen können, ein Stickstoffatom als Teil der cyclischen Struktur auf, mittels dessen er an den Triazinring der Struktur der Formel (I) gebunden ist. Ein nicht einschränkendes Beispiel eines solchen Triazinderivats ist eine Verbindung der folgenden Formel (III):

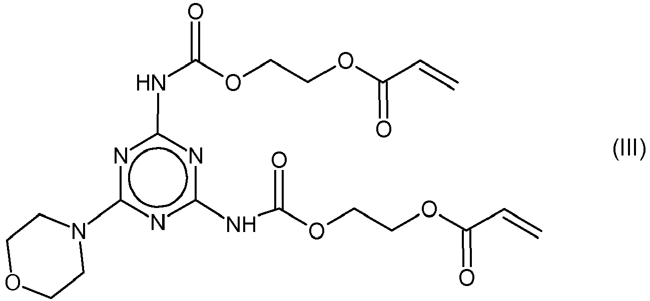

In einer weiteren bevorzugten Ausgestaltung der Erfindung ist jedes der an den Triazinring der Struktur der Formel (I) gebundenen Stickstoffatome in der gleichen Weise substituiert ist, so dass sich strukturell symmetrische Triazinderivate ergeben.

Die erfindungsgemäßen Triazinderivate lassen sich beispielsweise nach folgendem Verfahren herstellen: Ausgehend von modifizierten Melaminen erfolgt zunächst eine Umsetzung mit organischen Carbonaten. Anschließend kann bei Bedarf die Alkoxyterminierten Carbonatstruktur durch Alkohole und/oder primäre bzw. sekundäre Amine und/oder Aminoalkohole ausgetauscht werden. Werden mehrwertige Alkohole, Amine oder Aminoalkohole eingesetzt, kann so der Aufbau linearer oder verzweigter mehrkerniger Triazinderivatoligomere bzw. Triazinderivatpolymere erfolgen. Die eingesetzten Alkohole, Amine oder Aminoalkohole bestimmen dabei den Rest R² der Struktur der Formel (I).

Beispiele für vorteilhaft einzusetzende Alkohole sind Methanol, Ethanol, Propanol, Isopropanol, Butanol, Isobutanol, Pentanol, Hexanol, Benzylalkohol, Phenol, Ethylenglykolmonomethylether (2-Methoxyethanol), Ethylhexylalkohol, Dodecylalkohol und Stearylalkohol.

Weitere Beispiele für vorteilhaft einzusetzende substituierte Alkohole sind 2-Hydroxyethylacrylat, 2-Hydroxypropylacrylat, 4-Hydroxybutylacrylat, 6-Hydroxyhexylacrylat, Diethylenglykolmonoacrylat, Triethylenglykolmonoacrylat, Dipropylenglykolmonoacrylat, Tripropylenglykolmonoacrylat, Trimethylolpropandiacrylat, Pentaerythritdiacrylat, Pentaerythrittriacrylat, 2-Hydroxyethylmethacrylat, 2-Hydroxypropylmethacrylat, 4-Hydroxybutylmethacrylat, 6-Hydroxyhexylmethacrylat, Diethylenglykolmonomethacrylat, Triethylenglykolmonomethacrylat, Dipropylenglykolmonomethacrylat, Tripropylenglykolmonomethacrylat, Trimethylolpropandimethacrylat, Pentaerythritdimethacrylat, Pentaerythrit-trimethacrylat und/oder 4-Hydroxybutylvinylether.

Beispiele für vorteilhaft einzusetzende mehrwertige Alkohole sind Ethylenglykol, Butandiol, Octandiol, Dodecandiol und Octadecandiol, Glykol, Trimethylolpropan, Pentaerythrit, Polyethylenglykole mit Molmassen von 200 bis 5000, Polypropylenglykole mit Molmassen von 200 bis 5000, Polytetrahydrofurane mit Molmassen von 200 bis 5000, Polyesterdiole auf Basis gesättigter Dicarbonsäuren wie Terephthalsäure, Isophthalsäure, Naphthalindicarbonsäure Maleinsäure, Fumarsäure und/oder Itakonsäure und Diolen wie Ethylenglykol, Butandiol, Neopentylglykol und/oder Hexandiol.

Beispiele für vorteilhaft einzusetzende Amine und Diamine sind Ethylamin, Diethylamin, Propylamin, Dipropylamin, Isopropylamin, Diisopropylamin, Butylamin, Dibutylamin, Hexylamin, Hexamethylendiamin, Propylendiamin, Butylendiamin und/oder Ethylendiamin.

Beispiele für vorteilhaft einzusetzende Aminoalkohole sind Aminoethanol, Aminopropanol, Aminoethoxyethanol, Diethanolamin, Triethanolamin, Triisopropanolamin, Diisopropanolamin und Isopropanolamin.

Vorzugsweise weist zumindest einer der Reste R¹, R⁵, R⁶, R⁷ und R⁸ eine C₁-C₁₈-Alkylgruppe auf, um besonders günstige Eigenschaften des Triazinderivats hinsichtlich Schmelzpunkt und Viskosität zu bewirken. Dies trifft vorzugsweise insbesondere für die Reste R¹, R⁵ und R⁷ zu.

Zur Bereitstellung von modifizierten Melaminmonocarbamten bzw. modifizierten Melamindicarbamten ist es vorteilhaft, wenn dem Rest R⁶ und/oder dem Rest R⁸ nur die Bedeutung Q² , nicht jedoch die Bedeutung CO-A-R², -CO-A-R⁶¹ oder -CO-A-R⁸¹ zukommt.

Die erfindungsgemäßen Triazinderivate sind in Abhängigkeit der eingeführten Gruppen vorzugsweise strahlungshärtend bzw. unter Strahlung polymerisierbar. Dabei geschieht die Härtung/Polymerisierung vorzugsweise radikalisch beispielsweise über Doppelbindungen oder sauer beispielsweise über Epoxidstrukturen. Je nach Anzahl der hinsichtlich einer Polymerisierung vorhandenen reaktiven Gruppen können entsprechende Triazinderivate beispielsweise zur Vernetzung eingesetzt werden.

Als zur Härtung bzw. Polymerisierung vorteilhafte Strahlungstypen haben sich elektromagnetische Strahlung, beispielsweise in Form von UV-Strahlung, und Partikelstrahlung, beispielsweise in Form von Elektronenstrahlung, erwiesen.

Die Triazinderivate können in einer weiteren bevorzugten Ausgestaltung der Erfindung auch mit beispielsweise hydroxyterminierten Polyestern durch Kondensation und/oder mit Epoxiden durch Addition zur Reaktion gebracht werden und eignen sich deshalb auch als Vernetzer solcher Stoffklassen. Eine Initiierung einer entsprechenden Polymerisationsreaktion oder auch anderer Polymerisationsreaktionen kann beispielsweise thermisch und/oder chemisch erfolgen.

Durch die Einstellbarkeit des Verhältnisses von Hydrophobie zu Hydrophilie über das Substitutionsmusters können die erfindungsgemäßen Triazinderivate an Verbindungen anderer Stoffklassen hervorragend angepasst werden. Gerade durch die Senkung der Wasserstoffbrückenbindungen an den (insbesondere -CO-A-R²-, -CO-A-R⁶¹- oder -CO-AR⁸¹-substituierten) Aminogruppen des Melamins wird eine verbesserte Löslichkeit erreicht, so dass auch höhermolekulare Verbindungen, wie Oligomere und Polymere, besser in anderen Substanzen bzw. Stoffen gelöst werden können. In Oligomeren oder Polymeren sind einzelne Triazinderivatmonomere (bzw. substituierte Melaminkerne bzw. substituierte Triazinkerne) miteinander kovalent verbunden.

Gegenstand der Erfindung sind auch Mischungen, insbesondere reaktive Mischungen, der erfindungsgemäßen Triazinderivate mit mindestens einer Mischsubstanz, wobei hierzu Harnstoffharz, Harnstoff-Melaminharz, Melaminharz, Melamin-Phenolharz, Phenolharz, Polyester, Polyacrylat, Polyurethan, Epoxid, Polyether und/oder ein Gemisch dieser Substanzen zählen. Als reaktive Mischungen sind dabei solche Mischungen zu verstehen, bei denen die Triazinderivate mit der mindestens einen Mischsubstanz bei einer Anwendung, insbesondere während einer Aushärtung, miteinander reagieren können.

Die erfindungsgemäßen Triazinderivate und die oben definierten Mischungen können vorzugsweise als Vernetzer und/oder Bindemittel in Beschichtungsmassen, Farben und Lacken Verwendung finden. Insbesondere durch die definierte Einstellung der Schmelzpunkte der Triazinderivate sind Pulverlacke ein besonders bevorzugtes Einsatzgebiet. Aber auch die hohe Anzahl an vernetzbaren bzw. vernetzenden Gruppen in den Triazinderivaten bietet Vorteile bezüglich der Eigenschaften der Triazinderivate, insbesondere hinsichtlich Härte und Kratzfestigkeit.

Eine weitere vorteilhafte Verwendung der Triazinderivate und der oben definierten Mischungen ist deren Einsatz als Bindemittel für Verbundwerkstoffe (Compounds) und Formmassen, in Schäumen, in Fasern und zusammen mit weiteren Stoffen (insbesondere Synergisten) in Brandschutzausrüstungen.

Eine weitere vorteilhafte Verwendung der Triazinderivate und der oben definierten Mischungen ist deren Einsatz als Vernetzer und/oder Coreaktand bei der Herstellung eines Laminats zur Verbesserung der Oberflächeneigenschaften des Laminats. Als Oberflächeneigenschaften sind dabei insbesondere die Kratzfestigkeit, Härte und Haptik des Laminats zu nennen. Die Laminate können bevorzugt für die holzverarbeitende Industrie bereitgestellt werden.

Gegenstand der Erfindung ist auch ein Verfahren zur Herstellung Triazinderivaten der allgemeinen Formel (I) 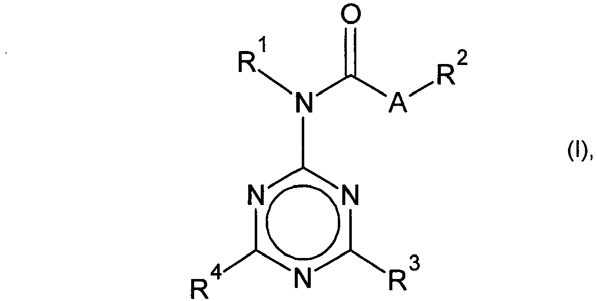 wobei
- **R³** Q¹ oder einen mit dem Stickstoffatom an den Triazinring der Struktur der Formel (I) gebundenen Rest der Formel **R⁵-**N**-R⁶** bedeutet, wobei
   - Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte
   - Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet;
- **R⁴** Q¹ oder einen mit dem Stickstoffatom an den Triazinring der Struktur der Formel (I) gebundenen Rest der Formel **R⁷-**N**-R⁸** bedeutet,
- **R¹, R⁵,** und **R⁷** unabhängig voneinander H oder Q² bedeuten, wobei
   - Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
- **A** -O-, -NH-, -N**R²-,** -N**R⁶²-** oder -N**R⁸²-** bedeutet,
- **R⁶** und **R⁸** unabhängig voneinander H, Q² -CO-**A-R²,** -CO-**A-R⁶¹,** -CO**-A-R⁸¹** bedeuten,
- **R², R⁶¹, R⁶², R⁸¹** und **R⁸²** unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) 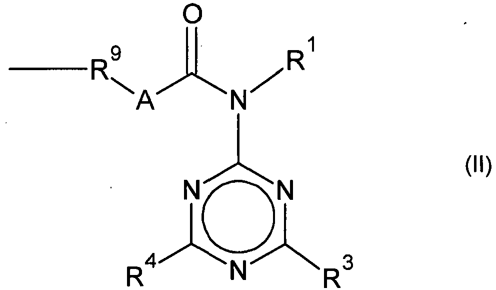 bedeuten,
   - wobei **R⁹** Q² bedeutet,
- mit der Maßgabe, dass, sofern **R³ R⁵-**N**-R⁶** und **R⁴ R⁷-**N**-R⁸** bedeuten,
   - mindestens einer der Reste **R¹, R⁵,** und **R⁷** Q² bedeutet und an ein Stickstoffatom gebunden ist, das kovalent mit dem Rest -CO**-A-R²,** -CO**-A-R⁶¹** oder -CO**-A-R⁸¹** verbunden ist, oder
   - zumindest die Reste **R⁵** und **R⁶** einerseits oder die Reste **R⁷** und **R⁸** andererseits jeweils Q² bedeuten oder
   - zumindest einer der Reste **R⁵** oder **R⁶** Q² bedeutet und **R⁷** und **R⁸** gleichzeitig beide H bedeuten,
      oder Mischungen davon. Dieses Verfahren zeichnet sich aus durch
      A) eine Umsetzung eines Triazinderivats der allgemeinen Formel (IX) 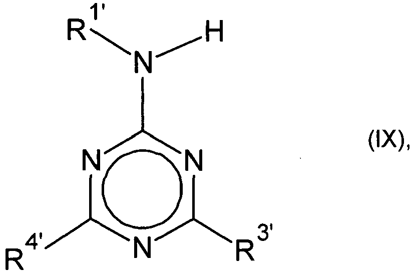 wobei
- **R^{3'}** Q¹ oder einen mit seinem zentralen Stickstoffatom an den Triazinring der Struktur der Formel (IX) gebundenen Rest der Formel **R^{5'}-**N**-R^{6'}** bedeutet, wobei
   Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O-unterbrochen sein kann, bedeutet,
- **R**^{**4**'} Q¹ oder einen mit dem Stickstoffatom an den Triazinring der Struktur der Formel (IX) gebundenen Rest der Formel **R^{7'}-**N**-R^{8'}** bedeutet,
- **R^{1'}**, **R^{5'},** und **R^{7'}** unabhängig voneinander H oder Q² bedeuten, wobei
   - Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder-OC(O)O- unterbrochen sein kann, bedeutet,
- **R^{6'}** und **R^{8'}** unabhängig voneinander H, Q², **-**CO**-A-R²,** -CO**-A-R⁶¹** oder **-**CO**-A-R⁸¹** bedeuten,
- **A**-O-, -NH- oder -N**R⁶²-** oder -N**R⁸²-**bedeutet,
- **R⁶¹, R⁶², R⁸¹** und **R⁸²** unabhängig voneinander Q² , einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) 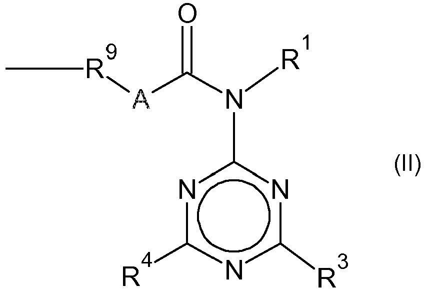 bedeuten,
- wobei **R⁹** Q² bedeutet,
   mit Phosgen und
   B) eine weitere Umsetzung des in Schritt A) gebildeten Carbamoylchlorids des Triazinderivats mit einem substituierten oder nicht substituierten Alkohol der allgemeinen Formel **R²-**OH, einem substituierten oder nicht substituierten mehrwertigen Alkohol der allgemeinen Formel **R²**-(OH)ₙ mit n ≥ 2, einem Amin der allgemeinen Formel **R²**-NH₂ oder **R²-**NH**-R²⁰,** einem Aminoalkohol der allgemeinen Formel OH-**R²**-NH₂ oder **R²**-NH-**R²⁰**-OH, einem Diamin der allgemeinen Formel **R²**-(NH₂)₂ oder einen Rest der allgemeinen Formel (II),
- wobei **R²** und **R²⁰** unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) bedeuten.

Die Umsetzung des in Schritt A) gebildeten Carbamoylchlorids des Triazinderivats mit einem Alkohol, Amin, Diamin oder Aminoalkohol erfolgt vorzugsweise sofort, das heißt ohne Isolierung des Zwischenprodukts.

Die optionalen Substituenten des eingesetzten Alkohols, Amins, Diamins oder Aminoalkohols sind vorzugsweise eine oder mehrere weitere OH- und/oder NH- und/oder NH₂-Gruppen. So können beispielsweise Alkohole, Amine, Diamine oder Aminoalkohole, bei denen R² und/oder R²⁰ unabhängig voneinander Q2 bedeutet und die eine oder mehrere weitere OH- und/oder NH- und/oder NH₂-Gruppen aufweisen, eingesetzt werden.

Hinsichtlich bevorzugt einzusetzender Alkohole, Amine und Aminoalkohole wird auf die obigen Erläuterungen in Bezug auf ein weiteres mögliches Herstellungsverfahren verwiesen, die auch für das hier beschriebene Verfahren zutreffen. Hinsichtlich weiterer bevorzugter Bedeutungen für die jeweiligen Reste wird auf die obigen Erläuterungen zu den erfindungsgemäßen Triazinderivaten verwiesen, die in analoger Weise auch für das herstellungsverfahren zutreffen.

Nach Abschluss der Reaktion kann das gebildete Triazincarbamat durch Umsetzung mit einem substituierten oder nicht substituierten Alkohol der allgemeinen Formel R²-OH, einem substituierten oder nicht substituierten mehrwertigen Alkohol der allgemeinen Formel R²-(OH)ₙ mit n ≥ 2, einem Amin der allgemeinen Formel R²-NH₂ oder R²-NH-R²⁰, einem Aminoalkohol der allgemeinen Formel OH-R²-NH₂ oder R²-NH-R²⁰-OH, einem Diamin der allgemeinen Formel R²-(NH₂)₂ weiter modifiziert werden. Die Reste R² und R²⁰ können dabei unabhängig voneinander die oben angegebenen Bedeutungen haben, wobei vorzugsweise ein Reaktand mit einem anderen R² als bei der Herstellung der Triazinderivate eingesetzt wird.

Auf diese Weise kann der Rest R² des Triazinderivats ausgetauscht werden (ähnlich zu einer Umetherung) Werden hierbei mehrwertige Alkohole, Amine oder Aminoalkohole eingesetzt, kann so der Aufbau linearer oder verzweigter mehrkerniger Triazinderivatoligomere bzw. Triazinderivatpolymere erfolgen.

Die Umsetzung des Triazinderivats mit Phosgen in Schritt A) des Verfahrens erfolgt vorzugsweise in einer Inertgas-Atmosphäre bei Temperaturen von ca. -20°C bis ca. +80°C, wobei vorteilhaft ein Überschuss an Phosgen eingesetzt wird. Insbesondere wird zunächst (zum Mischen des Triazinderivatedukts mit Phosgen) bei niedrigen Temperaturen im Bereich von ca. -10 °C bis ca. +30 °C, insbesondere ca. 0 °C bis ca. +20 °C oder bis ca. +25°C gearbeitet und danach die Temperatur zur eigentlichen Reaktion auf ca. 40°C bis ca. 70°C, insbesondere ca. 50°C bis ca. 60°C, erhöht.

Nach Beendigung der Reaktion wird der Überschuss an Phosgen durch Erhitzen auf Temperaturen von ca. 100°C bis ca. 200°C, insbesondere ca. 110°C bis 150 °C und ganz besonders ca. 120°C bis ca. 140°C ausgetrieben.

Die Umsetzung des gebildeten Carbamoylchlorides des Triazinderivats mit dem Alkohol R²-OH oder R²-(OH)ₙ mit n ≥ 2, dem Amin R²-NH₂ oder R²-NH-R²⁰, dem Aminoalkohol OH-R²-NH₂ oder R²-NH-R²⁰-OH oder dem Diamin R²-(NH₂)₂ erfolgt bei einer Temperatur von ca. +50 °C bis ca. +150 °C, insbesondere bei ca. 60°C bis ca. 100°C, ganz besonders bei ca. 70°C bis ca. 90°C, vorzugsweise in einer Inertgas-Atmosphäre. Der dabei gebildete Chlorwasserstoff kann durch einen Inertgas-Strom ausgetrieben werden.

Die Reaktion wird insbesondere in Lösung durchgeführt, wobei vorzugsweise Chlorbenzol, Nitrobenzol, Dichlorbenzol und Dioxan als Lösungsmittel zum Einsatz kommen.

Die nachfolgenden Beispiele beschreiben die Herstellung von Ausführungsbeispielen der erfindungsgemäßen Triazinderivate.

### Beispiel 1:

0,5 mol N,N',N"-Trimethylmelamin werden mit 12 Moläquivalenten Dimethylcarbonat in einem Kolben vorgelegt und auf 70°C erwärmt. 11 Moläquivalente Natriummethanolat werden in 2 I Methanol gelöst und über einen Tropftrichter in die Mischung eingetropft. Nach der Zugabe wird 120 min bei Rückfluss gerührt und anschließend abgekühlt und mit wässriger Phosphorsäure neutralisiert. Das ausfallende Produkt wird abgesaugt und getrocknet. Nach Analyse mittels HPLC wird ein Gehalt an 94 % N,N',N"-Trimethyl-N,N',N"-tris(methoxycarbonyl)-melamin der nachfolgenden Formel (IV) ermittelt:

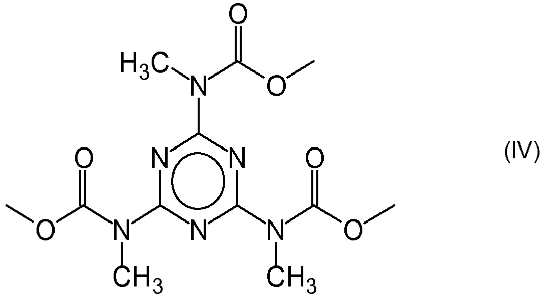

### Beispiel 2:

0,5 mol N,N-Dimethylmelamin werden mit 8 Moläquivalenten Dimethylcarbonat in einem Kolben vorgelegt und auf 70°C erwärmt. 7 Moläquivalente Natriummethanolat werden in 2 I Methanol gelöst und über einen Tropftrichter in die Mischung eingetropft. Nach der Zugabe wird 90 min bei Rückfluss gerührt und anschließend abgekühlt und mit wässriger Phosphorsäure neutralisiert. Das ausfallende Produkt wird abgesaugt und getrocknet. Nach Analyse mittels HPLC wird ein Gehalt an 98 % N,N-Dimethyl-N',N"-di-(methoxycarbonyl)-melamin der nachfolgenden Formel (V) ermittelt:

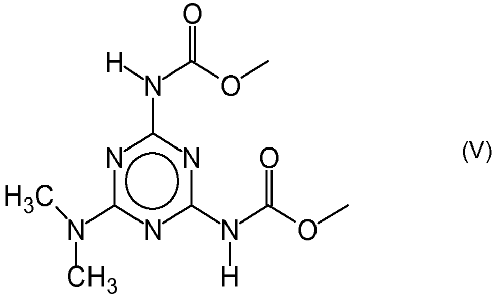

### Beispiel 3:

0,5 mol N-Monomethylmelamin wird mit 12 Moläquivalenten Dimethylcarbonat in einem Kolben vorgelegt und auf 70°C erwärmt. 11 Moläquivalente Natriummethanolat werden in 2 I Methanol gelöst und über einen Tropftrichter in die Mischung eingetropft. Nach der Zugabe wird 120 min bei Rückfluss gerührt und anschließend abgekühlt und mit wässriger Phosphorsäure neutralisiert. Das ausfallende Produkt wird abgesaugt und getrocknet. Nach Analyse mittels HPLC wird ein Gehalt an 92 % N-Monomethyl-N,N',N"-tris(methoxycarbonyl)-melamin der nachfolgenden Formel (VI) ermittelt:

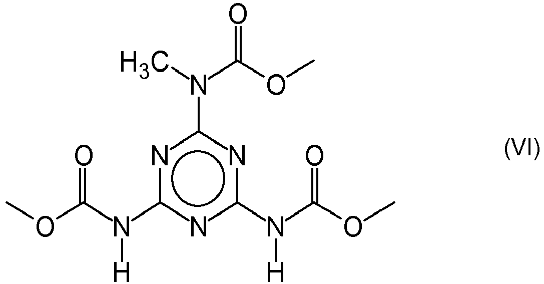

### Beispiel 4:

0,5 mol Produkt aus Beispiel 1 (N,N',N"-Trimethyl-N,N',N"-tris(methoxycarbonyl)-melamin) werden in 12 Moläquivalenten 2-Hydroxyethylacrylat in Gegenwart von 1 % Zirconacetylacetonat und 0,5 % Hydrochinonmonomethylether auf 100 °C erhitzt und 5 h gerührt. Anschließend wird das überschüssige Hydroxyethylacrylat abdestilliert. Der Rückstand enthält die gewünschte Zielverbindung (VII) zu 68%.

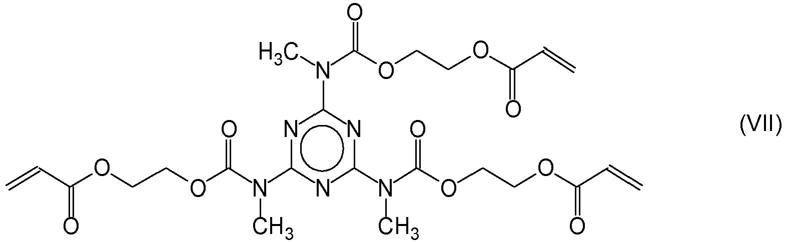

### Beispiel 5:

0,5 mol Produkt aus Beispiel 2 (N,N,N',N"-Tetramethyl-N',N"- bis(methoxycarbonyl)-melamin) werden in 8 Moläquivalenten 2-Hydroxyethylacrylat in Gegenwart von 1 % Zirconacetylacetonat und 0,5 % Hydrochinonmonomethylether auf 100 °C erhitzt und 5 h gerührt. Anschließend wird das überschüssige Hydroxyethylacrylat abdestilliert. Der Rückstand enthält die gewünschte Zielverbindung (VIII) zu 46%.

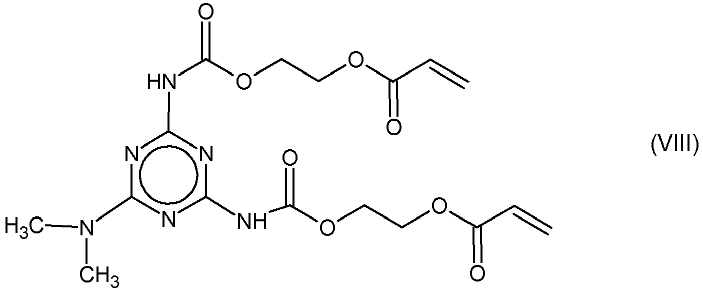

### Beispiel 6:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.51 g *N*,*N*,*N*',*N*"-Tetramethylmelamin (8.28 mmol) in 100 ml Chlorbenzol wurde in 30 min zugetropft, wobei die Temperatur auf 25 °C anstieg. Unter schwachem Phosgenstrom durch die Mischung wurde 1 h bei 25 °C und 1 h bei 60 °C gerührt. Es wurde eine Suspension des N-Methyl-N-Triazinyl-carbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 70 °C wurden 100 ml 2-Propanol (1.31 mol) in 15 min zugesetzt, wobei die Mischung klar wurde. Zur Vervollständigung der Reaktion wurde die Mischung unter aufrechtem (aufrecht erhaltenem) Stickstoffstrom 1 h bei 80 °C gerührt. Nach Abziehen des Lösungsmittel unter reduziertem Druck wurde ein zähes klares Öl erhalten, das sich nach Stehen unter reduziertem Druck in trockener Atmosphäre verfestigte.

Analysen mittels HPLC-MS, ¹H-NMR, ¹³C-NMR, and ESI-MS (pos. mode) ergaben, dass das Produkt aus den erwünschten Carbamaten 4-Dimethylamino-6-(isopropoxycarbonyl-methyl-amino)-1,3,5-triazin-2-yl)-methyl-carbaminsäureisopropylester (XII) und (4-Dimethylamino-6-methylamino-1,3,5-triazin-2-yl)-methyl-carbaminsäureisopropylester (X) bestand sowie geringe Mengen des Ausgangsstoffes *N,N,N',N"*-Tetramethylmelamin (XI) enthielt (siehe Tabelle 1).

**Tabelle 1: Ergebnis der Analyse der Produkte des Beispiels 6 mit HPLC/MS**

| Peak Nr. | [M+H]⁺ | Strukturformel | | Anteil in Flächen-% |
|---|---|---|---|---|
| 1 | 269.06 | 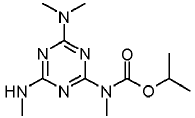 | (X) | 18.4% |
| 2 | 183.19 | 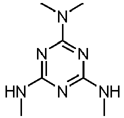 | (XI) | 23.8% |
| 3 | 355.3 | 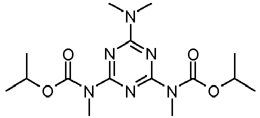 | (XII) | 57.8% |

| | | | | |
|---|---|---|---|---|
| "Flächen-%" bedeutet dabei hier und in den weiteren Beispielen die auf den jeweiligen Peak prozentual entfallende Fläche im HPLC-Chromatogramm. [M+H]⁺ gibt jeweils die ermittelte Masse der in dem Peak enthaltenen Substanz zuzüglich der Masse eines Protons in g/mol an. | | | | |

### Analysendaten für Peak Nr. 1:

¹H-NMR (200 MHz, CDCl₃): δ = 6.88 (s_{b}, 1H, -NH), 5.10 (p, *J* = 6.25 Hz, 1H, -C**H**-(CH₃)₂), 3.36 (s, 3H, >N-C**H**₃), 3.16 (s, 6H, -N(C**H**₃)₂), 2.97 (d, *J =* 4.92 Hz, 3H, -NH(C**H**₃)), 1.32 (d, *J* = 6.25 Hz, 6H, -CH-(C**H**₃)₂) ppm.

¹³C-NMR (APT puls program, 50 MHz, CDCl₃): *δ* = 164.7 (>**C**=O or =N-**C**=N-), 154.3 (>**C**=O or =N-**C**=N-), 164.7 (>**C**=O or =N-**C**=N-), 163.0 (>**C**=O or =N-**C**=N-),158.8 (>**C**=O or =N-**C**=N-), 154.3 (>**C**=O or =N-**C**=N-), 70.88 (-**C**H(CH₃)₂), 36.53 (-N(**C**H₃)₂), 33.91 (>N-**C**H₃), 27.78 (-NH-**C**H₃), 22.18 (-CH(**C**H₃)₂) ppm.

ESI-MS (c∼0.1 mg·cm⁻³, CHCl₃:*i*PrOH = 1:1, positive ion mode): *m*/*z* = 269.13 [M+H]⁺ TLC: R*_{f}*(ethyl acetate) = 0.67

### Analysendaten für Peak Nr. 3:

¹H-NMR (200 MHz, CDCl₃): *δ* = 5.10 (p, *J =* 6.26 Hz, 1H, -C**H**-(CH₃)₂), 3.42 (s, 3H, >N-C**H**₃ or -N(C**H**₃)₂), 3.20 (s, 3H, >N-C**H**₃ or -N(C**H**₃)₂), 1.33 (d, *J* = 6.26 Hz, 6H, -CH-(C**H**₃)₂) ppm.

¹³C-NMR (APT pulsprogram, 50 MHz, CDCl₃): *δ* = 161.1 (>**C**=O or =N-**C**=N-), 154.6 (>**C**=O or =N-**C**=N-), 154.4 (>**C**=O or =N-**C**=N-), 74.40 (-**C**H(CH₃)₂), 38.38 (>N-**C**H₃ or -N(**C**H₃)₂), 33.61 (>N-**C**H₃ or -N(**C**H₃)₂), 21.90 (-CH(**C**H₃)₂) ppm.

ESI-MS (c∼0.1 mg.cm⁻³, CHCl₃:*i*PrOH = 1:1, positive ion mode): *m*/*z* = 355.13 [M+H]⁺ TLC: R*_{f}*(ethyl acetate) = 0.82

### Beispiel 7:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.26 g *N,N,N"-*Trimethylmelamin (7.48 mmol) in 100 ml Chlorbenzol wurde in 30 min zugetropft, wobei die Temperatur auf 20 °C anstieg. Unter schwachem Phosgenstrom durch die Mischung wurde 1 h bei 20 °C und 1 h bei 60 °C gerührt. Es wurde eine Suspension des *N*-Methyl-*N-*Triazinyl-carbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 70 °C wurden 100 ml 2-Propanol (1.31 mol) in 15 min zugesetzt, wobei die Mischung klar wurde. Zur Vervollständigung der Reaktion wurde die Mischung unter aufrechtem Stickstoffstrom 1 h bei 80 °C gerührt. Nach Abziehen des Lösungsmittel unter reduziertem Druck wurde ein zähes klares Öl erhalten, das sich nach Stehen unter reduziertem Druck in trockener Atmosphäre verfestigte.

Analyse mittels HPLC-MS ergab (vgl. Tabelle 2), dass das Produkt aus den erwünschten Carbamaten [4,6-Bis-(methylamino)-1,3,5-triazin-2-yl]-methyl-carbaminsäure-isopropylester (XIII), [4-(lsopropoxycarbonyl-methylamino)-6-methylamino-1,3,5-triazin-2-yl]-methyl-carbaminsäure-isopropylester (XV) und [4,6-Bis-(isopropoxycarbonyl-methylamino)-1,3,5-triazin-2-yl]-methyl-carbaminsäure-isopropylester (XVI) bestand sowie geringe Mengen des Ausgangsstoffes *N,N',N"*-Trimethylmelamin (XIV) und von mehrkernigen Melaminderivaten (Nebenprodukten) enthielt.

**Tabelle 2: Ergebnis der Analyse der Produkte des Beispiels 7 mit HPLC/MS**

| Peak Nr. | [M+H]⁺ | Strukturformel | | Anteil in Flächen-% |
|---|---|---|---|---|
| 1 | 255,16 | 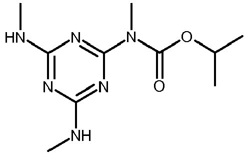 | (XIII) | 17,3 |
| 2 | 169,12 | 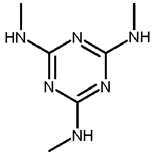 | (XIV) | 13,6 |
| 3 | 341,19 | 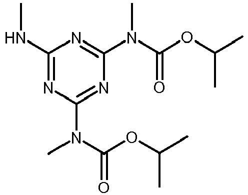 | (XV) | 22,8 |
| 4 | 427,23 | 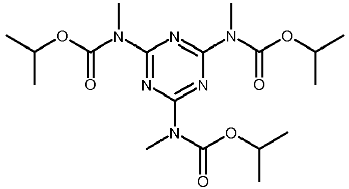 | (XVI) | 46,3 |

### Beispiel 8:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.25 g *N*,*N*',*N*"-Trimethylmelamin (7.43 mmol) in 100 ml Chlorbenzol wurde in 30 min zugetropft, wobei die Temperatur auf 20 °C anstieg. Unter schwachem Phosgenstrom durch die Mischung wurde 1 h bei 20 °C und 1 h bei 60 °C gerührt. Es wurde eine Suspension des N-Methyl-N-triazinylcarbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 80 °C wurden 3.33 g Phenol (35.4 mmol) in 15 min zugesetzt, wobei die Mischung klar wurde. Zur Vervollständigung der Reaktion wurde die Mischung unter aufrechtem Stickstoffstrom 1 h bei 80 °C gerührt. Nach Abziehen des Lösungsmittel unter reduziertem Druck wurde ein hochviskoses braunes Öl erhalten, das laut Analyse mit HPLC/MS die gewünschten Carbamate enthielt (vgl. Tabelle 3).

**Tabelle 3: Ergebnis der Analyse der Produkte des Beispiels 8 mit HPLC/MS**

| Peak Nr. | [M+H]⁺ | Strukturformel | Anteil in Flächen-% |
|---|---|---|---|
| 1 | 289,14 | 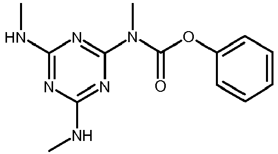 | 17,4 |
| 2 | 169,12 | 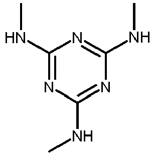 | 12,0 |
| 3 | 409,16 | 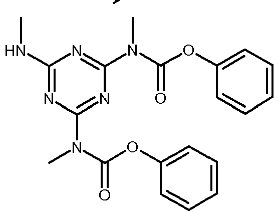 | 20,7 |
| 4 | 529,18 | 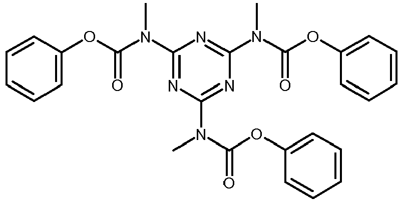 | 49,9 |

### Beispiel 9:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.25 g *N,N',N"-*Trimethylmelamin (7.43 mmol) in 100 ml Chlorbenzol wurde in 30 min zugetropft, wobei die Temperatur auf 20°C anstieg. Unter schwachem Phosgenstrom durch die Mischung wurde 1 h bei 20°C und 1 h bei 60°C gerührt. Es wurde eine Suspension des N-Methyl-N-triazinylcarbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 60 °C wurden 1,42 g Methanol (44.4 mmol) in 15 min zugesetzt, wobei die Mischung klar wurde. Zur Vervollständigung der Reaktion wurde die Mischung unter aufrechtem Stickstoffstrom 1 h bei 60 °C gerührt. Nach Abziehen des Lösungsmittels unter reduziertem Druck wurde ein zähes klares Öl erhalten, das sich nach Stehen unter reduziertem Druck in trockener Atmosphäre verfestigte und das laut Analyse mit HPLC/MS die gewünschten Carbamate enthielt (vgl. Tabelle 4).

**Tabelle 4: Ergebnis der Analyse der Produkte des Beispiels 9 mit HPLC/MS**

| Peak Nr. | [M+H]⁺ | Strukturformel | Anteil in Flächen-% |
|---|---|---|---|
| 1 | 227,13 | 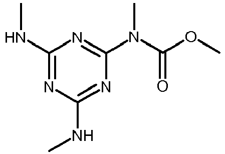 | 17,2 |
| 2 | 169,12 | 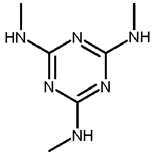 | 9,2 |
| 3 | 285,13 | 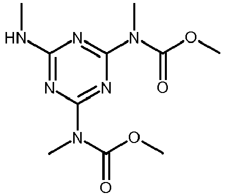 | 23,5 |
| 4 | 343,14 | 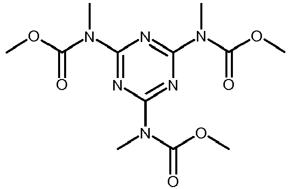 | 47,1 |

### Beispiel 10:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.50 g *N,N,N',N"-*Tetramethylmelamin (8.23 mmol) in 100 ml Chlorbenzol wurde in 30 min zugetropft, wobei die Temperatur auf 20°C anstieg. Unter schwachem Phosgenstrom durch die Mischung wurde 1 h bei 20 °C und 1 h bei 60°C gerührt. Es wurde eine Suspension des *N*-Methyl-*N-*triazinyl-carbamoylchlorides erhalten.

Bei 130°C wurde 15 min Stickstoff wurde durch die Mischung geleitet, um überschüssiges gelöstes Phosgen auszutreiben. Nach Abkühlen der Mischung auf 60°C wurden 1,58 g Methanol (49.4 mmol) in 15 min zugesetzt, wobei die Mischung klar wurde. Zur Vervollständigung der Reaktion wurde die Mischung unter aufrechtem Stickstoffstrom 1 h bei 60°C gerührt. Nach Abziehen des Lösungsmittel unter reduziertem Druck wurde ein zähes klares Öl erhalten, das sich nach Stehen unter reduziertem Druck in trockener Atmosphäre verfestigte und das laut Analyse mit HPLC/MS die gewünschten Carbamate enthielt (vgl. Tabelle 5).

**Tabelle 5: Ergebnis der Analyse der Produkte des Beispiels 10 mit HPLC/MS**

| Peak Nr. | [M+H]⁺ | Strukturformel | Anteil in Flächen-% |
|---|---|---|---|
| 1 | 241,14 | 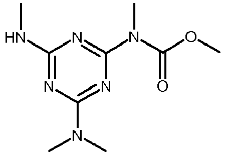 | 30,3 |
| 2 | 183,14 | 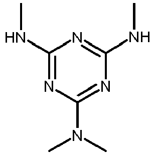 | 13,8 |
| 3 | 299,15 | 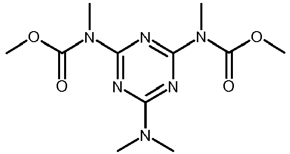 | 55,9 |

### Beispiel 11:

In einem Dreihals-Rundkolben (500 ml) mit Gaseinleitrohr, Innenthermometer, beheizbarem Tropftrichter und Rückflusskühler wurden 100 ml Chlorbenzol 30 min mit Stickstoff gespült und danach 10 min bei 0 °C mit Phosgen beladen. Eine Lösung von 1.25 g *N,N',N"-*Trimethylmelamin (7.43 mmol) in 100 ml Chlorbenzol wurde in 30 min zugetropft, wobei die Temperatur auf 20°C anstieg. Unter schwachem Phosgenstrom durch die Mischung wurde 1 h bei 20°C und 1 h bei 60°C gerührt. Es wurde eine Suspension des N-Methyl-N-triazinylcarbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 60 °C wurden 4,41 g 1-Oktanol (33,88 mmol) in 15 min zugesetzt, wobei die Mischung klar wurde. Zur Vervollständigung der Reaktion wurde die Mischung unter aufrechtem Stickstoffstrom 1 h bei 60 °C gerührt. Nach Abziehen des Lösungsmittel unter reduziertem Druck und der flüchtigen Bestandteile wurde ein zähes Öl erhalten, das laut Analyse mit HPLC/MS die gewünschten Carbamate enthielt (vgl. Tabelle 6).

**Tabelle 6: Ergebnis der Analyse der Produkte des Beispiels 11 mit HPLC/MS**

| Peak Nr. | [M+H]⁺ | Strukturformel | Anteil in Flächen-% |
|---|---|---|---|
| 1 | 325,24 | 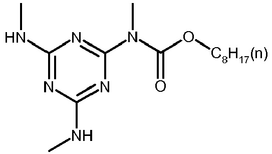 | 14,2 |
| 2 | 169,12 | 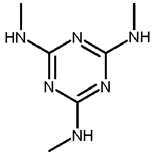 | 19,2 |
| 3 | 481,35 | 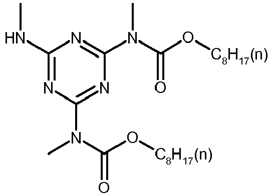 | 19,5 |
| 4 | 637,47 | 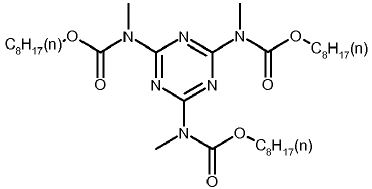 | 44,1 |

Die in den Strukturformeln der Tabelle 6 verwendete Bezeichnung C₈H₁₇(n) bedeutet dabei, dass es sich jeweils um einen n-Oktylrest (also einen linearen Oktylrest) handelt.

### Beispiel 12:

In einem Dreihals-Rundkolben (500 ml) mit Gaseinleitrohr, Innenthermometer, beheizbarem Tropftrichter und Rückflusskühler wurden 100 ml Nitrobenzol 30 min mit Stickstoff gespült und danach 10 min bei 0 °C mit Phosgen beladen. Eine Lösung von 1.25 g *N,N',N"-*Trimethylmelamin (7.43 mmol) in 100 ml Nitrobenzol wurde in 30 min zugetropft, wobei die Temperatur auf 20 °C anstieg. Unter schwachem Phosgenstrom durch die Mischung wurde 1 h bei 20 °C und 1 h bei 60 °C gerührt. Es wurde eine Suspension des N-Methyl-N-triazinylcarbamoylchlorides erhalten.

Bei 130 °C wurde 15 min Stickstoff wurde durch die Mischung geleitet, um überschüssiges gelöstes Phosgen auszutreiben. Nach Abkühlen der Mischung auf 60 °C wurden 5,95 g (2-Hydroxyethyl)-methacrylat (45,7 mmol; stabilisiert mit 0,3 % 2,2,6,6-Tetramethyl-piperidin-1-oxyl (TEMPO)) in 15 min zugesetzt, wobei die Mischung klar wurde. Zur Vervollständigung der Reaktion wurde die Mischung unter aufrechtem Stickstoffstrom 1 h bei 60 °C gerührt. Nach Abziehen des Lösungsmittel und der flüchtigen Bestandteile unter reduziertem Druck wurde ein dunkles Öl erhalten, das laut Analyse mit HPLC/MS die gewünschten Carbamate enthielt (vgl. Tabelle 7).

**Tabelle 7: Ergebnis der Analyse der Produkte des Beispiels 12 mit HPLC/MS**

| Peak Nr. | [M+H]⁺ | Strukturformel | Anteil in Flächen-% |
|---|---|---|---|
| 1 | 325 | 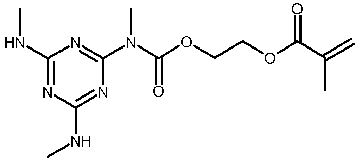 | 16,7 |
| 2 | 169,12 | 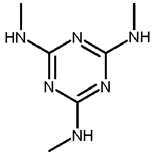 | 16,8 |
| 3 | 481 | 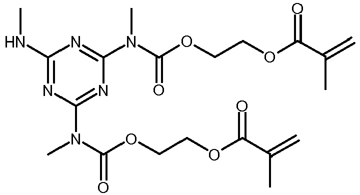 | 21,3 |
| 4 | 637 | 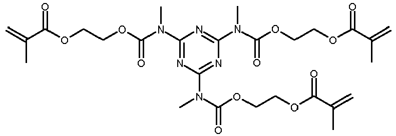 | 45,2 |

Die jeweiligen Reaktionsprodukte der vorgenannten Beispiele können analog zu den Beispielen 4 und 5 weiter umgesetzt werden. Dabei können die Reaktionsprodukte vor einer weiteren Umsetzung voneinander getrennt und gereinigt werden oder auch ohne vorherige Aufarbeitung weiter umgesetzt werden.

## Patentansprüche

1. Triazinderivate gemäß der allgemeinen Formel (la) 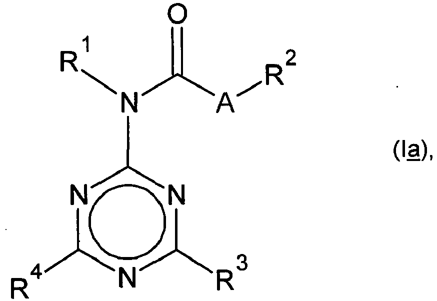 wobei
• **R³** Q¹ oder einen mit dem Stickstoffatom an den Triazinring der Struktur der Formel (la) gebundenen Rest der Formel **R⁵-**N**-R⁶** bedeutet, wobei
- Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
• **R⁴** einen mit dem Stickstoffatom an den Triazinring der Struktur der Formel (la) gebundenen Rest der Formel **R⁷-**N**-R⁸** bedeutet,
• **R¹, R⁵,** und **R⁷** unabhängig voneinander Q² bedeuten, wobei
- Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Neue Ansprüche_Mitteilung gemäß Regel 71(3) EPÜ
Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
• **A** -NH-, **-**N**R²-,** -N**R⁶²**- oder -N**R⁸²**- bedeutet,
• **R⁶,** Q² -CO-**A-R²,** -CO**-A-R⁶¹**, **-CO-A-R⁸¹** bedeutet,
• **R⁸** -CO-**A-R²,** -CO**-A-R⁶¹, CO-A-R⁸¹** bedeutet,
• **R², R⁶¹ , R⁶², R⁸¹** und **R⁸²** unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) 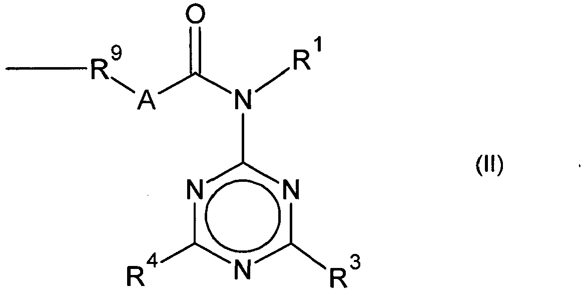 bedeuten,
- wobei **R⁹** Q² bedeutet,
• mit der Maßgabe, dass, sofern **R³ R⁵-**N**-R⁶** und **R⁴ R⁷-**N**-R⁸** bedeuten,
- mindestens einer der Reste **R¹, R⁵,** und **R⁷** Q² bedeutet und an ein Stickstoffatom gebunden ist, das kovalent mit dem Rest -CO-**A-R²,** -CO**-A-R⁶¹** oder -CO-**A-R⁸¹** verbunden ist, oder Neue Ansprüche_Mitteilung gemäß Regel 71(3) EPÜ
- zumindest die Reste **R⁵** und **R⁶** einerseits oder die Reste **R⁷** und **R⁸** andererseits jeweils Q² bedeuten
oder Mischungen davon.

2. Triazinderivate gemäß der allgemeinen Formel (Ib) 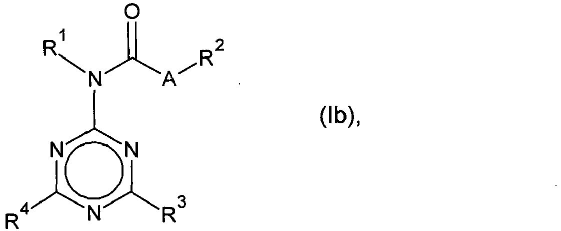 wobei
• **R³** Q¹ oder einen mit dem Stickstoffatom an den Triazinring der Struktur der Formel (Ib) gebundenen Rest der Formel **R⁵-**N**-R⁶** bedeutet, wobei
- Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent durch ein oder mehrere Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
• **R⁴** Q¹ oder einen mit dem Stickstoffatom an den Triazinring der Struktur der Formel (Ib) gebundenen Rest der Formel **R⁷-**N**-R⁸** bedeutet,
• **R¹, R⁵,** und **R⁷** unabhängig voneinander Q² bedeuten, wobei
- Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils durch ein oder mehrere Neue Ansprüche_Mitteilung gemäß Regel 71(3) EPÜ
Sauerstoffatome, Schwefelatome, substituierte und/oder unsubstituierte Stickstoffatome und/oder durch ein oder mehrere Gruppen des Typs -C(O)O-, -OC(O)-, -C(O)-, -NHC(O)O-, -OC(O)NH- und/oder -OC(O)O- unterbrochen sein kann, bedeutet,
• **A** -O- bedeutet,
• **R⁶** Q² -CO-**A-R²,** -CO-**A-R⁶¹,** -CO-**A-R⁸¹** bedeutet,
• **R⁸** Q², -CO-**A-R²,** -CO**-A-R⁶¹,** -CO-**A-R⁸¹** bedeutet,
• **R², R⁶¹, R⁶², R⁸¹** und **R⁸²** unabhängig voneinander einen Rest eines substituierten oder nicht substituierten Alkohols ausgewählt aus den Verbindungen 2-Hydroxyethylacrylat, 2-Hydroxypropylacrylat, 4-Hydroxybutylacrylat, 6-Hydroxyhexylacrylat, Diethylenglykolmonoacrylat, Triethylenglykolmonoacrylat, Dipropylenglykolmonoacrylat, Tripropylenglykolmonoacrylat, Trimethylolpropandiacrylat, Pentaerythritdiacrylat, Pentaerythrittriacrylat, 2-Hydroxyethylmethacrylat, 2-Hydroxypropylmethacrylat, 4-Hydroxybutylmethacrylat, 6-Hydroxyhexylmethacrylat, Diethylenglykolmonomethacrylat, Triethylenglykolmonomethacrylat, Dipropylenglykolmonomethacrylat, Tripropylenglykolmonomethacrylat, Trimethylolpropan-dimethacrylat, Pentaerythritdimethacrylat, Pentaerythrittrimethacrylat und/oder 4-Hydroxybutylvinylether, oder einen Rest der allgemeinen Formel (II) 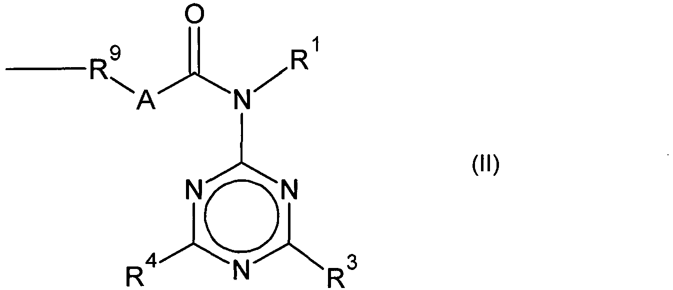 bedeuten,
- wobei **R⁹** Q² bedeutet, Neue Ansprüche_Mitteilung gemäß Regel 71(3) EPÜ
• mit der Maßgabe, dass, sofern **R³ R⁵-**N**-R⁶** und **R**⁴ **R⁷-**N**-R⁸** bedeuten,
- mindestens einer der Reste **R¹**, **R⁵,** und **R⁷** Q² bedeutet und an ein Stickstoffatom gebunden ist, das kovalent mit dem Rest -CO**-A-R²,** -CO-**A**-**R⁶¹** oder -CO-**A-R⁸¹** verbunden ist, oder
- zumindest die Reste **R⁵** und **R⁶** einerseits oder die Reste **R⁷** und **R⁸** andererseits jeweils Q² bedeuten,
oder Mischungen davon.

3. Triazinderivate nach Anspruch 1 oder 2, **dadurch gekennzeichnet, dass** die Reste **R², R⁶¹, R⁶², R⁸¹** und **R⁸²** identisch sind.

4. Triazinderivate nach einem der vorhergehenden Ansprüche, **dadurch gekennzeichnet, dass** sie eine einstellbare Viskosität und/oder ein einstellbares Schmelzverhalten und/oder eine einstellbare Kompatibilität gegenüber anderen Stoffen aufweisen.

5. Triazinderivate nach einem der vorhergehenden Ansprüche, **dadurch gekennzeichnet, dass** mindestens einer der Reste **R¹**, **R⁵, R⁶, R⁷** und **R⁸,** unabhängig von der Bedeutung der Reste **R³** und **R⁴,** Q² bedeutet.

6. Triazinderivate nach einem der vorhergehenden Ansprüche, **dadurch gekennzeichnet, dass** mindestens einer der Reste **R¹**, **R⁵, R⁶, R⁷** und **R⁸,** unabhängig von der Bedeutung der Reste **R³** und **R⁴,** Q² bedeutet und an ein Stickstoffatom gebunden ist, das kovalent mit dem Rest -CO-**A-R²,** -CO**-A-R⁶¹** oder -CO-**A-R⁸¹** verbunden ist.

7. Triazinderivate nach einem der vorhergehenden Ansprüche, **dadurch gekennzeichnet, dass** mindestens einer der Reste **R⁵, R⁶**, **R⁷** und **R⁸,** unabhängig von der Bedeutung der Reste **R³** und **R⁴,** Q² bedeutet.

8. Triazinderivate nach einem der vorhergehenden Ansprüche, **dadurch gekennzeichnet, dass** der cyclische Substituent ein Stickstoffatom als Teil der cyclischen Struktur aufweist, mittels dessen er an den Triazinring der Struktur der Formel (Ia,b) gebunden ist. Neue Ansprüche_Mitteilung gemäß Regel 71(3) EPÜ

9. Triazinderivate nach einem der vorhergehenden Ansprüche, **dadurch gekennzeichnet, dass** jedes der an den Triazinring der Struktur der Formel (Ia,b) gebundenen Stickstoffatome in der gleichen Weise substituiert ist.

10. Triazinderivate nach einem der Ansprüche 1 und 3 bis 9, **dadurch gekennzeichnet, dass R**², **R**⁶¹, **R**⁶², **R**⁸¹ und **R**⁸² unabhängig voneinander ein Rest von Methanol, Ethanol, Propanol, Isopropanol, Butanol, Isobutanol, Pentanol, Hexanol, Benzylalkohol, Phenol, Ethylenglykolmonomethylether, Ethylhexylalkohol, Dodecylalkohol und/oder Stearylalkohol sind.

11. Triazinderivate nach einem der Ansprüche 1 und 3 bis 10, **dadurch gekennzeichnet, dass R**², **R**⁶¹, **R**⁶², **R**⁸¹ und **R**⁸² unabhängig voneinander ein Rest der Verbindungen 2-Hydroxyethylacrylat, 2-Hydroxypropylacrylat, 4-Hydroxybutylacrylat, 6-Hydroxyhexylacrylat, Diethylenglykolmonoacrylat, Triethylenglykolmonoacrylat, Dipropylenglykolmonoacrylat, Tripropylenglykolmonoacrylat, Trimethylolpropandiacrylat, Pentaerythritdiacrylat, Pentaerythrittriacrylat, 2-Hydroxyethylmethacrylat, 2-Hydroxypropylmethacrylat, 4-Hydroxybutylmethacrylat, 6-Hydroxyhexylmethacrylat, Diethylenglykolmonomethacrylat, Triethylenglykolmonomethacrylat, Dipropylenglykolmonomethacrylat, Tripropylenglykolmonomethacrylat, Trimethylolpropandimethacrylat, Pentaerythritdimethacrylat, Pentaerythrittrimethacrylat und/oder 4-Hydroxybutylvinylether, sind; unabhängig voneinander ein Rest von Ethylenglykol, Butandiol, Neopentylglykol, Hexandiol Octandiol, Dodecandiol, Octadecandiol, Glykol, Trimethylolpropan, Pentaerythrit, von Polyethylenglykolen mit Molmassen von 200 bis 5000, von Polypropylenglykolen mit Molmassen von 200 bis 5000, von Polytetrahydrofuranen mit Molmassen von 200 bis 5000 und/oder von Polyesterdiolen auf der Basis gesättigter Dicarbonsäuren wie Terephthalsäure, Isophthalsäure, Naphthalindicarbonsäure Maleinsäure, Fumarsäure und/oder Itakonsäure sind; unabhängig voneinander ein Rest von Ethylamin, Diethylamin, Propylamin, Dipropylamin, Isopropylamin, Diisopropylamin, Butylamin, Dibutylamin, Hexylamin, Hexamethylendiamin, Propylendiamin, Butylendiamin und/oder Ethylendiamin sind; oder unabhängig voneinander ein Rest von Aminoethanol, Aminopropanol, Aminoethoxyethanol, Diethanolamin, Triethanolamin, Triisopropanolamin, Diisopropanolamin und/oder Isopropanolamin sind. Neue Ansprüche_Mitteilung gemäß Regel 71(3) EPÜ

12. Triazinderivate nach einem der vorhergehenden Ansprüche, **dadurch gekennzeichnet, dass** zumindest einer der Reste **R**¹, **R**⁵, **R**⁶, **R**⁷ und **R**⁸ eine C₁-C₁₈-Alkylgruppe aufweist.

13. Triazinderivate nach einem der vorhergehenden Ansprüche, **dadurch gekennzeichnet, dass** dem Rest **R**⁶ und/oder dem Rest **R**⁸ nur die Bedeutung Q², nicht jedoch die Bedeutung CO-A-**R**², -CO-**A**-**R**⁶¹ oder -CO-**A**-**R**⁸¹ zukommt.

14. Mischung der Triazinderivate nach einem der vorhergehenden Ansprüche mit mindestens einem Harnstoffharz, Harnstoff-Melaminharz, Melaminharz, Melamin-Phenolharz, Phenolharz, Polyester, Polyacrylat, Polyurethan, Epoxid, Polyether und/oder einem Gemisch davon.

15. Verwendung der Triazinderivate nach einem der Ansprüche 1 bis 13 oder der Mischung nach Anspruch 14 als Vernetzer und/oder als Bindemittel in Beschichtungsmassen, Lacken und Farben, als Bindemittel für Verbundwerkstoffe und Formmassen, in Schäumen, in Fasern und zusammen mit weiteren Stoffen in Brandschutzausrüstungen, als Vernetzer und/oder Coreaktand bei der Herstellung eines Laminats zur Verbesserung der Oberflächeneigenschaften des Laminats

16. Verfahren zur Herstellung von Triazinderivaten nach einem der Ansprüche 1 bis 13
**gekennzeichnet durch**
A) eine Umsetzung eines Triazinderivats der allgemeinen Formel (IX) 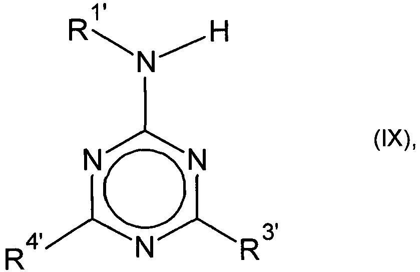 wobei
• **R**^{3'} **Q**¹ oder einen mit seinem zentralen Stickstoffatom an den Triazinring der Struktur der Formel (IX) gebundenen Rest der Formel **R**^{5'}-N-**R**^{6'} bedeutet, wobei
- Q¹ ein lineares oder verzweigtes C₁-C₅₀-Alkyl oder einen cyclischen Substituenten in Form eines C₅-C₂₀-Cycloalkyls, eines C₅-C₂₀-Aryls, eines C₁-C₂₀-alkylsubstituierten C₅-C₂₀-Aryls, eines C₂-C₂₀-alkenylsubstituierten C₅-C₂₀-Aryls oder eines Imids von cyclischen gesättigten oder ungesättigten Carbonsäuren, wobei das C₁-C₅₀-Alkyl oder der cyclische Substituent **durch** ein oder mehrere Sauerstoffatome, Schwefelatome, substituierte und/oder unsubstituierte Stickstoffatome und/oder **durch** ein oder mehrere Gruppen des Typs -C(O)O-, -OC(O)-, -C(O)-, -NHC(O)O-, -OC(O)NH- und/oder - OC(O)O- unterbrochen sein kann, bedeutet,
• **R**^{4'} Q¹ oder einen mit dem Stickstoffatom an den Triazinring der Struktur der Formel (IX) gebundenen Rest der Formel **R**^{7'}-N-**R**^{8'} bedeutet,
• **R**^{1'}, **R**^{5'}, und **R**^{7'} unabhängig voneinander Q² bedeuten, wobei
- Q² jeweils lineares oder verzweigtes C₁-C₅₀-Alkyl, C₅-C₂₀-Cycloalkyl, C₅-C₂₀-Aryl, C₁-C₂₀-alkylsubstituiertes C₅-C₂₀-Aryl, C₂-C₂₀-Alkenyl oder C₂-C₂₀-alkenylsubstituiertes C₅-C₂₀-Aryl, das jeweils **durch** ein oder mehrere Sauerstoffatome, Schwefelatome, substituierte und/oder unsubstituierte Stickstoffatome und/oder **durch** ein oder mehrere Gruppen des Typs - C(O)O-, -OC(O)-, -C(O)-, -NHC(O)O-, -OC(O)NH- und/oder -OC(O)O-unterbrochen sein kann, bedeutet,
• **R**^{6'} H, Q², -CO-A-**R**⁶¹ oder -CO-A-**R**⁸¹ bedeutet,
• **R**^{8'} H, Q2, CO-A-**R**⁶¹ oder -CO-A-**R**⁸¹ bedeutet,
• **A** -O-, -NH- oder -N**R**⁶²- oder -N**R**⁸²-bedeutet,
• **R**⁶¹, **R**⁶², **R**⁸¹ und **R**⁸² unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) 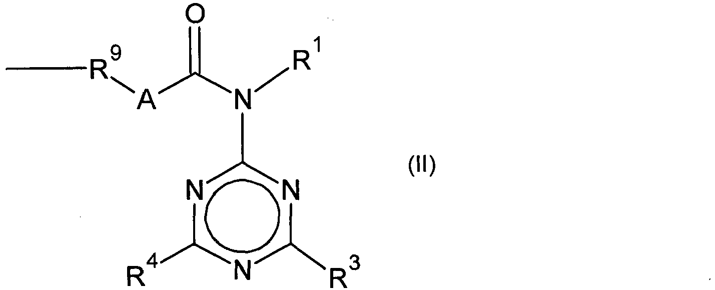 bedeuten,
• wobei **R**⁹ Q² bedeutet,
mit Phosgen und
B) eine weitere Umsetzung des in Schritt A) gebildeten Carbamoylchlorids des Triazinderivats mit einem substituierten oder nicht substituierten Alkohol der allgemeinen Formel **R**²-OH einem substituierten oder nicht substituierten mehrwertigen Alkohol der allgemeinen Formel **R**²-(OH)ₙ mit n ≥ 2, einem Amin der allgemeinen Formel **R**²-NH₂ oder **R**²-NH-**R**²⁰, einem Aminoalkohol der allgemeinen Formel OH-**R**²-NH₂ oder **R**²-NH-**R**²⁰-OH, einem Diamin der allgemeinen Formel **R**²-(NH₂)₂ oder einen Rest der allgemeinen Formel (II),
• wobei **R**² und **R**²⁰ unabhängig voneinander Q², einen Rest eines substituierten oder nicht substituierten Alkohols, einen Rest eines substituierten oder nicht substituierten mehrwertigen Alkohols, einen Rest eines Amins, einen Rest eines Aminoalkohols, einen Rest eines Diamins oder einen Rest der allgemeinen Formel (II) bedeutet.

## Claims

1. Triazine derivates according to the general formula (Ia) 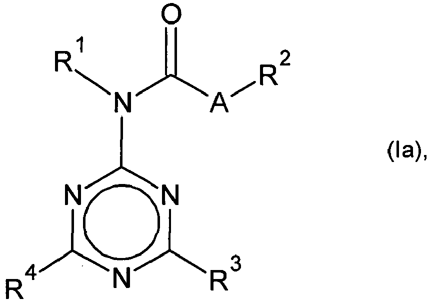 wherein
• **R**³ means Q¹ or a moiety of the formula **R**⁵-N-**R**⁶ connected with the nitrogen atom to the triazine ring of the structure of formula (Ia), wherein
- Q¹ means a linear or branched C₁-C₅₀-alkyl or a cyclic substituent in form of a C₅-C₂₀-cycloalkyl, a C₅-C₂₀-aryl, a C₁-C₂₀-alkyl substituted C₅-C₂₀-aryl, a C₂-C₂₀-alkenyl substituted C₅-C₂₀-aryl or of an imide of a cyclic saturated or unsaturated carboxylic acid, wherein the C₁-C₅₀-alkyl or the cyclic substituent can be interrupted by one or multiple oxygen atoms, sulphur atoms, substituted and/or unsubstituted nitrogen atoms and/or by one or multiple groups of the type -C(O)O-, -OC(O)-, -C(O)-, - NHC(O)O-, -OC(O)NH- and/or -OC(O)O-,
• **R**⁴ means Q¹ or a moiety of the formula **R**⁷-N-**R**⁸ connected with the nitrogen atom to the triazine ring of the structure of the formula (Ia),
• **R**¹, **R**⁵, and **R**⁷ mean independently from each other Q², wherein
- Q² means in each case a linear or branched C₁-C₅₀-alkyl, C₅-C₂₀-cycloalkyl, C₅-C₂₀-aryl, C₁-C₂₀-alkyl substituted C₅-C₂₀-aryl, C₂-C₂₀-alkenyl or C₂-C₂₀-alkenyl substituted C₅-C₂₀-aryl, that in each case can be interrupted by one or multiple oxygen atoms, sulphur atoms, substituted and/or unsubstituted nitrogen atoms and/or by one or multiple groups of the type -C(O)O-, -OC(O)-, -C(O)-, -NHC(O)O-, -OC(O)NH- and/or -OC(O)O-,
• A means -NH-, -N**R**²-, -N**R**⁶²- or -N**R**⁸²- ,
• **R**⁶ means Q² -CO-**A**-**R**², -CO-**A**-**R**⁶¹, -CO-**A**-**R**⁸¹,
• **R**⁸ means -CO-**A**-**R**², -CO-**A**-**R**⁶¹, -CO-**A**-**R**⁸¹
• **R**², **R**⁶¹, **R**⁶², **R**⁸¹, and **R**⁸² mean independent from each other Q², a moiety of a substituted or non-substituted alcohol, a moiety of a substituted or unsubstituted polyvalent alcohol, a moiety of an amine, a moiety of an amino alcohol, a moiety of a diamine or a moiety of the general formula (II) 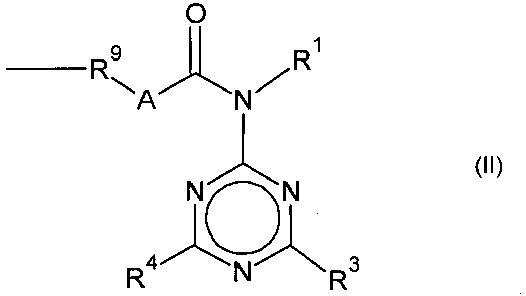
- wherein **R**⁹ means Q²,
• with the stipulation that in case **R**³ means **R**⁵-N-**R**⁶ and **R**⁴ means **R**⁷-N-**R**⁸,
- at least one of the moieties **R**¹, **R**⁵, and **R**⁷ means Q² and is bound to a nitrogen atom that is covalently connected to the moiety -CO-**A**-**R**², -CO-**A**-**R**⁶¹ or -CO-**A**-**R**⁸¹ , or
- at least the moieties **R**⁵ and **R**⁶ on the one hand and the moieties **R**⁷ and **R**⁸ on the other hand mean in each case Q²
or mixtures thereof.

2. Triazine derivates according to the general formula (Ib) 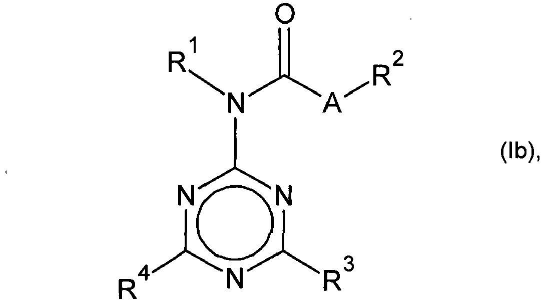 wherein
• **R**³ means Q¹ or a moiety of the formula **R**⁵-N-**R**⁶ connected with the nitrogen atom to the triazine ring of the structure of formula (Ib), wherein
- Q¹ means a linear or branched C₁-C₅₀-alkyl or a cyclic substituent in form of a C₅-C₂₀-cycloalkyl, a C₅-C₂₀-aryl, a C₁-C₂₀-alkyl substituted C₅-C₂₀-aryl, a C₂-C₂₀-alkenyl substituted C₅-C₂₀-aryl or of an imide of a cyclic saturated or unsaturated carboxylic acid, wherein the C₁-C₅₀-alkyl or the cyclic substituent can be interrupted by one or multiple oxygen atoms, sulphur atoms, substituted and/or unsubstituted nitrogen atoms and/or by one or multiple groups of the type -C(O)O-, -OC(O)-, -C(O)-, - NHC(O)O-, -OC(O)NH- and/or -OC(O)O-,
• **R**⁴ means Q¹ or a moiety of the formula **R**⁷-N-**R**⁸ connected with the nitrogen atom to the triazine ring of the structure of the formula (Ib),
• **R**¹, **R**⁵, and **R**¹ mean independently from each other Q², wherein
- Q² means in each case a linear or branched C₁-C₅₀-alkyl, C₅-C₂₀-cycloalkyl, C₅-C₂₀-aryl, C₁-C₂₀-alkyl substituted C₅-C₂₀-aryl, C₂-C₂₀-alkenyl or C₂-C₂₀-alkenyl substituted C₅-C₂₀-aryl, that in each case can be interrupted by one or multiple oxygen atoms, sulphur atoms, substituted and/or unsubstituted nitrogen atoms and/or by one or multiple groups of the type -C(O)O-, -OC(O)-, -C(O)-, -NHC(O)O-, -OC(O)NH- and/or -OC(O)O-,
• **A** means -O-,
• **R**⁶ means Q², -CO-**A**-**R**², -CO-**A**-**R**⁶¹, -CO-**A**-**R**⁸¹,
• **R**⁸ means Q², -CO-**A**-**R**², -CO-**A**-**R**⁶¹, -CO-**A**-**R**⁸¹,
• **R**², **R**⁶¹, **R**⁶², **R**⁸¹ and **R**⁸² mean independent from each a moiety of a substituted or non-substituted alcohol selected from the compounds 2-hydroxyethylacrylate, 2-hydroxypropylacrylate, 4-hydroxybutylacrylate, 6-hydroxyhexyl-acrylate, diethylenglycolmonoacrylate, triethylenglycolmonoacrylate, dipropylenglycolmonoacrylate, tripropylenglycolmonoacrylate, trimethylolpropandiacrylate, pentaerythritdiacrylate, pentaerythrittriacrylate, 2-hydroxyethylmethacrylate, 2-hydroxypropylmethacrylate, 4-hydroxybutylmethacrylate, 6-hydroxyhexylmethacrylate, diethylenglycolmonomethacrylate, triethylenglycolmonomethacrylate, dipropylen-glycolmonomethacrylate, tripropylenglycolmonomethacrylate, trimethylol-propandimethacrylate, pentaerythritdimethacrylate, pentaerythrittrimethacrylate and/or 4-hydroxybutylvinylether or a moiety of the general formula (II) 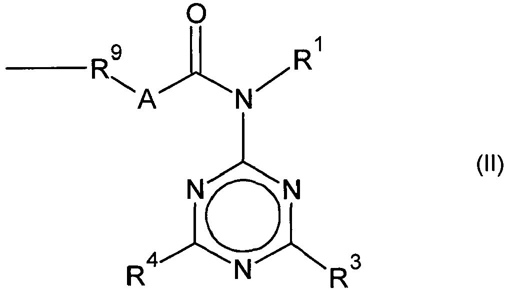 wherein **R**⁹ means Q²,
• with the stipulation that in case **R**³ means **R**⁵-N-**R**⁶ and **R**⁴ means **R**⁷-N-**R**⁸,
- at least one of the moieties **R**¹, **R**⁵, and **R**⁷ means Q² and is bound to a nitrogen atom that is covalently connected to the moiety -CO-**A**-**R**², -CO-**A**-**R**⁶¹ or -CO-**A**-R⁸¹ , or
- at least the moieties **R**⁵ and **R**⁶ on the one hand and the moieties **R⁷** and **R**⁸ on the other hand mean in each case Q²
or mixtures thereof.

3. Triazine derivates according to claim 1 or 2, **characterized in that,** the moieties **R**², **R**⁶¹, **R**⁶², **R**⁸¹ and **R**⁸² are identical.

4. Triazine derivatives according to according to one of the preceding claims, **characterized in that,** that they have an adjustable viscosity and/or an adjustable melting behaviour and/or an adjustable compatibility to other compounds.

5. Triazine derivates according to one of the preceding claims, **characterized in that,** that at least one of the moieties **R**¹, **R**⁵, **R**⁶, **R**⁷ and **R**⁸ mean Q² independent on the meaning of the moieties **R**³ and **R**⁴.

6. Triazine derivates according to one of the preceding claims, **characterized in that,** at least one of the moieties **R**¹, **R**⁵, **R**⁶, **R**⁷ and **R**⁸ mean Q² independent on the meaning of the moieties **R**³ and **R**⁴ and are bound to a nitrogen atom that is covalently linked to the moiety -CO-**A**-**R**², -CO-**A**-**R**⁶¹ or -CO-**A**-**R**⁸¹.

7. Triazine derivates according to one of the preceding claims, **characterized in that,** that at least one of the moieties **R**⁵, **R**⁶, **R**⁷ and **R**⁸ mean Q² independent on the meaning of the moieties **R**³ and **R**⁴.

8. Triazine derivates according to one of the preceding claims, **characterized in that,** the cyclic substituent has a nitrogen atom as part of the cyclic structure via which it is bound to the triazine ring of the structure of the formula (Ia,b).

9. Triazine derivates according to one of the preceding claims, **characterized in that,** each of the nitrogen atoms bound to the triazine ring of the structure of the formula (la, b) is substituted in the same manner.

10. Triazine derivates according to one of the claims 1 and 3 to 9, **characterized in that, R**², **R**⁶¹, **R**⁶², **R**⁸¹ and **R**⁸² are independent from each other a moiety of methanol, ethanol, propanol, isopropanol, butanol, isobutanol, pentanol, hexanol, benzylalcohol, phenol, ethylenglycolmonomethylether, ethylhexylalcohol, dodecylalcohol and/or stearylalcohol.

11. Triazine derivates according to one of the claims 1 and 3 to 10, **characterized in that, R**², **R**⁶¹, **R**⁶², **R**⁸¹ and **R**⁸² are independently from each other a moiety of the compound 2-hydroxyethylacrylate, 2-hydroxypropylacrylate, 4-hydroxybutylacrylate, 6-hydroxyhexyl-acrylate, diethylenglycolmonoacrylate, triethylenglycolmonoacrylate, dipropylenglycol-monoacrylate, tripropylenglycolmonoacrylate, trimethylolpropandiacrylate, pentaerythritdiacrylate, pentaerythrittriacrylate, 2-hydroxyethylmethacrylate, 2-hydroxypropylmethacrylate, 4-hydroxybutylmethacrylate, 6-hydroxyhexylmethacrylate, diethylenglycol-monomethacrylate, triethylenglycolmonomethacrylate, dipropylenglycolmonometh-acrylate, tripropylenglycolmonomethacrylate, trimethylolpropandimethacrylate, pentaerythritdimethacrylate, pentaerythrittrimethacrylate and/or 4-hydroxybutylvinylether; are independently from each other a moiety of ethylenglycol, butandiol, neopentylglycol, hexandiol, octandiol, dodecandiol, octadecandiol, glycol, trimethylolpropane, pentaerythrit, of polyethylenglycoles with molar masses of 200 to 5000, of polypropylenglycoles with molar masses of 200 to 5000, of polytetrahydrofuranes with molar masses of 200 to 5000 and/or of polyesterdioles on the basis of saturated dicarboxylic acids like terephthalic acid, isophthalic acid, naphthalindicarboxylic acid, maleinic acid, fumaric acid and/or itakonic acid; are independently from each other a moiety of ethylamine, diethylamine, propylamine, dipropylamine, isopropylamine, diisopropylamine, butylamine, dibutylamine, hexylamine, hexamethylendiamine, propylendiamine, butylendiamine and/or ethylendiamine; or are independently from each other a moiety of aminoethanol, aminopropanol, aminoethoxyethanol, diethanolamine, triethanolamine, triisopropanol-amine, diisopropanolamine and/or isopropanolamine.

12. Triazine derivates according to one of the preceding claims, **characterized in that,** at least on of the moieties **R**¹, **R**⁵, **R**⁶, **R**⁷ and **R**⁸ have a C₁-C₁₈-alkyl group.

13. Triazine derivates according to one of the preceding claims, **characterized in that,** the moiety **R**⁶ and/or the moiety **R**⁸ have only the meaning of Q², but not the meaning of CO-A-**R**², -CO-**A**-**R**⁶¹ or -CO-**A-R**⁸¹.

14. Composition of the triazine derivatives according to one of the preceding claims with at least one urea resin, urea-melamine resin, melamine resin, melamine-phenol-resin, phenol resin, polyester, polyacrylate, polyurethane, epoxide, polyether and/or mixture thereof.

15. Use of the triazine derivatives according to one of the claims 1 to 13 or the composition according to claim 14 as cross linking agent and/or as binder in coating compounds, lacquers and paints, as a binder for composites and moulding compositions, in foams, in fibers and fire protection equipment together with further substances, as cross linking agent and/or co-reactant in the production of a laminate for improving the surface properties of the laminate.

16. Method for the production of triazine derivates according to one of the claims 1 to 13, **characterized by**
A) conversion of a triazine derivative according to the general formula (IX) 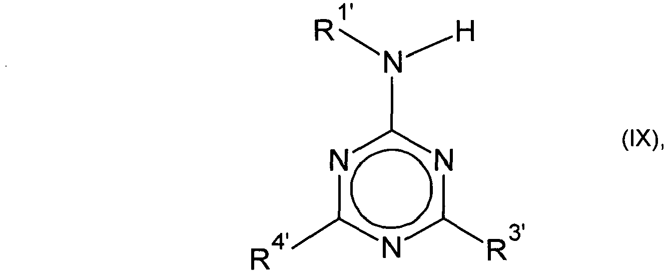 whereby
• **R**^{3'} means Q¹ or a moiety of the formula **R**^{5'}-N-**R**^{6'} bound with its central nitrogen atom to the triazine ring of the structure of the formula (IX), wherein
- Q¹ means a linear or branched C₁-C₅₀-alkyl or a cyclic substituent in form of a C₅-C₂₀-cycloalkyl, a C₅-C₂₀-aryl, a C₁-C₂₀-alkyl substituted C₅-C₂₀-aryl, a C₂-C₂₀-alkenyl substituted C₅-C₂₀-aryl or an imide of cyclic saturated or unsaturated carboxylic acids, wherein the C₁-C₅₀-alkyl or the cyclic substituent can be interrupted by one or multiple oxygen atoms, sulphur atoms, substituted and/or unsubstituted nitrogen atoms and/or by one or multiple groups of the type -C(O)O-, -OC(O)-, -C(O)-, -NHC(O)O-, -OC(O)NH- and/or -OC(O)O-,
• **R**^{4'} means Q¹ or a moiety of the formula **R**⁷-N-**R**^{8'} bound with the nitrogen atom to the triazine ring of the structure of the formula (IX),
• **R**^{1'}, **R**^{5'}, and **R**^{7'} independently from each other mean Q², wherein
- Q² means in each case linear or branched C₁-C₅₀-alkyl, C₅-C₂₀-cycloalkyl, C₅-C₂₀-aryl, C₁-C₂₀-alkyl substituted C₅-C₂₀-aryl, C₂-C₂₀-alkenyl or C₂-C₂₀-alkenyl substituted C₅-C₂₀-aryl, which can be interrupted in each case by one or multiple oxygen atoms, sulphur atoms, substituted and/or unsubstituted nitrogen atoms and/or by one or multiple groups of the type -C(O)O-, -OC(O)-, -C(O)-, -NHC(O)O-, -OC(O)NH- and/or -OC(O)O-,
• **R**^{6'} means H, Q², -CO-A-**R**⁶¹ or -CO-A-**R**⁸¹,
• **R**^{8'} means H, Q², -CO-A-**R**⁶¹ or -CO-A-**R**⁸¹,
• A means -O-, -NH- or -N**R**⁶²- or -N**R**⁸²-,
• **R**⁶¹, **R**⁶², **R**⁸¹ and **R**⁸² mean independently from each other Q², a moiety of a substituted or unsubstituted alcohol, a moiety of the substituted or unsubstituted polyvalent alcohol, a moiety of an amine, a moiety of an amino alcohol, a moiety of a diamine or a moiety of the general formula (II) 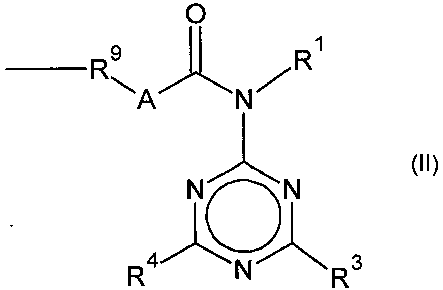 ,
• wherein **R**⁹ means Q²,
with phosgen and
B) a further conversion of the carbamoylchloride of the triazine derivative formed in step A) with a substituted or unsubstituted alcohol of the general formula **R**²-OH, a substituted or unsubstituted polyvalent alcohol of the general formula **R**²-(OH)ₙ with n ≥ 2, an amine of the general formula **R**²-NH₂ or **R**²-NH-**R**²⁰, an amino alcohol of the general formula OH-**R**²-NH₂ or **R**²-NH-**R**²⁰-OH, a diamine of the general formula **R**²-(NH₂)₂ or a moiety of the general formula (II),
• whereby **R**² and **R**²⁰ mean independently from each other Q², a moiety of a substituted or unsubstituted alcohol, a moiety of a substituted or unsubstituted polyvalent alcohol, a moiety of an amine, a moiety of an amino alcohol, a moiety of a diamine or a moiety of the general formula (II).

## Revendications

1. Dérivés de triazine selon la formule générale (Ia) 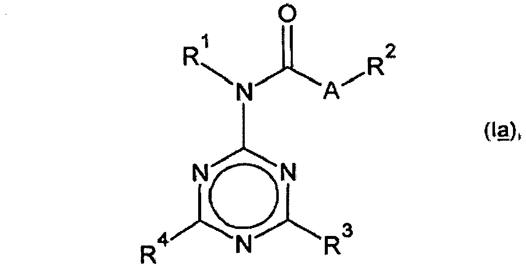 dans laquelle
- R³ représente Q¹ ou un radical lié à l'atome d'azote sur le cycle triazine de structure de formule (Ia), ayant la formule R⁵-N-R⁶, dans lequel
-- Q¹ représente un alkyle en C₁-C₅₀ linéaire ou ramifié ou un substituant cyclique sous la forme d'un cycloalkyle en C₅-C₂₀, d'un aryle en C₅-C₂₀, d'un aryle en C₅-C₂₀ substitué par un alkyle en C₁-C₂₀, d'un aryle en C₅-C₂₀ substitué par un alcényle en C₂-C₂₀ ou d'un imide d'acides carboxyliques cycliques saturés ou insaturés, où l'alkyle en C₁-C₅₀ ou le substituant cyclique peut être interrompu par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-,-OC(O)NH- et/ou -OC(O)O-,
- R⁴ représente un radical lié à l'atome d'azote sur le cycle triazine de structure de formule (Ia), ayant la formule R⁷-N-R⁸,
- R¹, R⁵ et R⁷ représentent, indépendamment les uns des autres, Q², dans lequel
-- Q² représente respectivement un alkyle en C₁-C₅₀ linéaire ou ramifié, un cycloalkyle en C₅-C₂₀, un aryle en C₅-C₂₀, un aryle en C₅-C₂₀ substitué par un alkyle en C₁-C₂₀, un alcényle en C₂-C₂₀ ou un aryle en C₅-C₂₀ substitué par un alcényle en C₂-C₂₀, qui peut être interrompu à chaque fois par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-, -OC(O)NH-et/ou -OC(O)O-,
- A représente NH-, -NR²-, -NR⁶²- ou NR⁸²-,
- R⁶ représente Q² -CO-A-R², -CO-A-R⁶¹, -CO-A-R⁸¹,
- R⁸ représente -CO-A-R², -CO-A-R⁶¹, -CO-A-R⁸¹,
- R², R⁶¹, R⁶², R⁸¹ et R⁸² représentent, indépendamment les uns des autres, Q², un radical d'un alcool substitué ou non substitué, un radical d'un alcool polyvalent substitué ou non substitué, un radical d'une amine, un radical d'un aminoalcool, un radical d'une diamine ou un radical de formule générale (II) 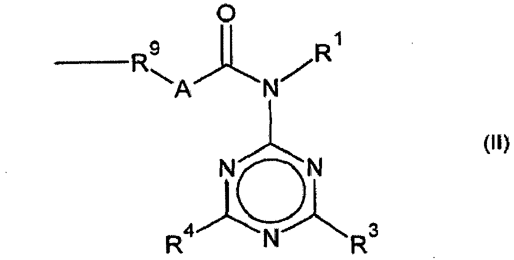
-- dans laquelle R⁹ représente Q²,
- à condition que, si R³ représente R⁵-N-R⁶ et R⁴ représente R⁷-N-R⁸,
-- au moins l'un des radicaux R¹, R⁵ et R⁷ représente Q² et est relié à un atome d'azote, qui est lié de manière covalente au radical -CO-A-R², -CO-A-R⁶¹ ou -CO-A-R⁸¹, ou
-- au moins les radicaux R⁵ et R⁶ d'une part ou les radicaux R⁷ et R⁸ d'autre part représentent respectivement Q²,
ou des mélanges de ceux-ci.

2. Dérivés de triazine selon la formule générale (Ib) 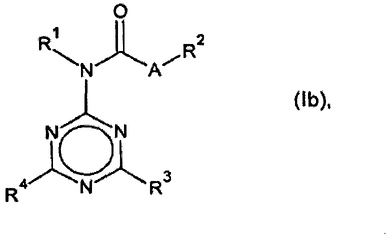 dans laquelle
- R³ représente Q¹ ou un radical lié à l'atome d'azote sur le cycle triazine de structure de formule (Ib), ayant la formule R⁵-N-R⁶, dans lequel
-- Q¹ représente un alkyle en C₁-C₅₀ linéaire ou ramifié ou un substituant cyclique sous la forme d'un cycloalkyle en C₅-C₂₀, d'un aryle en C₅-C₂₀, d'un aryle en C₅-C₂₀ substitué par un alkyle en C₁-C₂₀, d'un aryle en C₅-C₂₀ substitué par un alcényle en C₂-C₂₀ ou d'un imide d'acides carboxyliques cycliques saturés ou insaturés, où l'alkyle en C₁-C₅₀ ou le substituant cyclique peut être interrompu par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-,-OC(O)NH- et/ou -OC(O)O-,
- R⁴ représente Q¹ ou un radical lié à l'atome d'azote sur le cycle triazine de structure de formule (Ib), ayant la formule R⁷-N-R⁸,
- R¹, R⁵ et R⁷ représentent, indépendamment les uns des autres, Q², dans lequel
-- Q² représente respectivement un alkyle en C₁-C₅₀ linéaire ou ramifié, un cycloalkyle en C₅-C₂₀, un aryle en C₅-C₂₀, un aryle en C₅-C₂₀ substitué par un alkyle en C₁-C₂₀, un alcényle en C₂-C₂₀ ou un aryle en C₅-C₂₀ substitué par un alcényle en C₂-C₂₀, qui peut être interrompu à chaque fois par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-, -OC(O)NH- et/ou -OC(O)O-,
- A représente -O-,
- R⁶ représente Q², -CO-A-R², -CO-A-R⁶¹, -CO-A-R⁸¹,
- R⁸ représente Q², -CO-A-R², -CO-A-R⁶¹, -CO-A-R⁸¹,
- R², R⁶¹, R⁶², R⁸¹ et R⁸² représentent, indépendamment les uns des autres, un radical d'un alcool substitué ou non substitué, choisi parmi les composés acrylate de 2-hydroxyéthyle, acrylate de 2-hydroxypropyle, acrylate de 4-hydroxybutyle, acrylate de 6-hydroxyhexyle, monoacrylate de diéthylène glycol, monoacrylate de triéthylène glycol, monoacrylate de dipropylène glycol, monoacrylate de tripropylène glycol, diacrylate de triméthylolpropane, diacrylate de pentaérythrite, triacrylate de pentaérythrite, méthacrylate de 2-hydroxyéthyle, méthacrylate de 2-hydroxypropyle, méthacrylate de 4-hydroxybutyle, méthacrylate de 6-hydroxyhexyle, monométhacrylate de diéthylène glycol, monométhacrylate de triéthylène glycol, monométhacrylate de dipropylène glycol, monométhacrylate de tripropylène glycol, diméthacrylate de triméthylolpropane, diméthacrylate de pentaérythrite, triméthacrylate de pentaérythrite, et/ou éther de 4-hydroxybutylvinyle, ou un radical de formule générale (II) 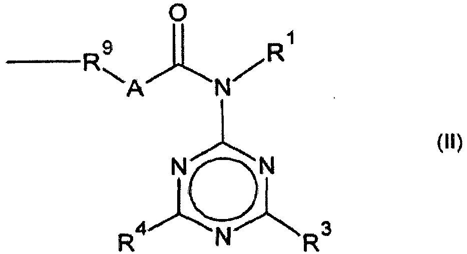
-- dans laquelle R⁹ représente Q²,
- à condition que, si R³ représente R⁵-N-R⁶ et R⁴ représente R⁷-N-R⁸,
-- au moins l'un des radicaux R¹, R⁵ et R⁷ représente Q² et est relié à un atome d'azote, qui est lié de manière covalente au radical -CO-A-R², -CO-A-R⁶¹ ou -CO-A-R⁸¹, ou
-- au moins les radicaux R⁵ et R⁶ d'une part ou les radicaux R⁷ et R⁸ d'autre part représentent respectivement Q²,
ou des mélanges de ceux-ci.

3. Dérivés de triazine selon la revendication 1 ou 2, **caractérisés en ce que** les radicaux R², R⁶¹, R⁶², R⁸¹ et R⁸² sont identiques.

4. Dérivés de triazine selon l'une des revendications précédentes, **caractérisés en ce qu'**ils présentent une viscosité ajustable et/ou un comportement de fusion ajustable et/ou une compatibilité ajustable par rapport aux autres substances.

5. Dérivés de triazine selon l'une des revendications précédentes, **caractérisés en ce qu'**au moins l'un des radicaux R¹, R⁵, R⁶, R⁷ et R⁸ représente, indépendamment de la signification des radicaux R³ et R⁴, Q².

6. Dérivés de triazine selon l'une des revendications précédentes, **caractérisés en ce qu'**au moins l'un des radicaux R¹, R⁵, R⁶, R⁷ et R⁸ représente, indépendamment de la signification des radicaux R³ et R⁴, Q² et est relié à un atome d'azote, qui est lié de manière covalente au radical -CO-A-R², -CO-A-R⁶¹ ou -CO-A-R⁸¹.

7. Dérivés de triazine selon l'une des revendications précédentes, **caractérisés en ce qu'**au moins l'un des radicaux R⁵, R⁶, R⁷ et R⁸ représente, indépendamment de la signification des radicaux R³ et R⁴, Q².

8. Dérivés de triazine selon l'une des revendications précédentes, **caractérisés en ce que** le substituant cyclique présente un atome d'azote en tant que partie de la structure cyclique, au moyen de laquelle il est lié sur le cycle triazine de la structure de formules (Ia,b).

9. Dérivés de triazine selon l'une des revendications précédentes, **caractérisés en ce que** chacun des atomes d'azote liés au cycle triazine de la structure de formules (Ia,b) est substitué de la même manière.

10. Dérivés de triazine selon l'une des revendications 1 et 3 à 9, **caractérisés en ce que** R², R⁶¹, R⁶², R⁸¹ et R⁸² sont, indépendamment les uns des autres, un radical de méthanol, d'éthanol, de propanol, d'isopropanol, de butanol, d'isobutanol, de pentanol, d'hexanol, d'alcool benzylique, de phénol, d'éther monométhylique d'éthylène glycol, d'alcool éthylhexylique, d'alcool dodécylique et/ou d'alcool stéarylique.

11. Dérivés de triazine selon l'une des revendications 1 et 3 à 10, **caractérisés en ce que** R², R⁶¹, R⁶², R⁸¹ et R⁸² sont, indépendamment les uns des autres, un radical des composés acrylate de 2-hydroxyéthyle, acrylate de 2-hydroxypropyle, acrylate de 4-hydroxybutyle, acrylate de 6-hydroxyhexyle, monoacrylate de diéthylène glycol, monoacrylate de triéthylène glycol, monoacrylate de dipropylène glycol, monoacrylate de tripropylène glycol, diacrylate de triméthylolpropane, diacrylate de pentaérythrite, triacrylate de pentaérythrite, méthacrylate de 2-hydroxyéthyle, méthacrylate de 2-hydroxypropyle, méthacrylate de 4-hydroxybutyle, méthacrylate de 6-hydroxyhexyle, monométhacrylate de diéthylène glycol, monométhacrylate de triéthylène glycol, monométhacrylate de dipropylène glycol, monométhacrylate de tripropylène glycol, diméthacrylate de triméthylolpropane, diméthacrylate de pentaérythrite, triméthacrylate de pentaérythrite, et/ou éther de 4-hydroxybutylvinyle ; indépendamment les uns des autres, un radical d'éthylène glycol, de butanediol, de néopentyle glycol, d'hexanediol, d'octanediol, de dodécanediol, d'octadécanediol, de glycol, de triméthylolpropane, de pentaérythrite, de polyéthylène glycols ayant des poids moléculaires de 200 à 5000, de polypropylène glycols ayant des poids moléculaires de 200 à 5000, de polytétrahydrofuranes ayant des poids moléculaires de 200 à 5000 et/ou de polyesterdiols à base d'acides dicarboxyliques saturés comme l'acide téréphtalique, l'acide isophtalique, l'acide naphtalène dicarboxylique, l'acide maléique, l'acide fumarique et/ou l'acide itaconique ; indépendamment les uns des autres, un radical d'éthylamine, de diéthylamine, de propylamine, de dipropylamine, d'isopropylamine, de düsopropylamine, de butylamine, de dibutylamine, d'hexylamine, d'hexaméthylène diamine, de propylène diamine, de butylène diamine, et/ou d'éthylène diamine ; ou chacun indépendamment les uns des autres un radical d'aminoéthanol, d'aminopropanol, d'aminoéthoxyéthanol, de diéthanolamine, de triéthanolamine, de triisopropanolamine, de diisopropanolamine et/ou d'isopropanolamine.

12. Dérivés de triazine selon l'une des revendications précédentes, **caractérisés en ce qu'**au moins l'un des radicaux R¹, R⁵, R⁶, R⁷ et R⁸ présente un groupe alkyle en C₁-C₁₈.

13. Dérivés de triazine selon l'une des revendications précédentes, **caractérisés en ce que** le radical R⁶ et/ou le radical R⁸ prennent seulement la signification Q², mais pas la signification -CO-A-R², -CO-A-R⁶¹ ou -CO-A-R⁸¹.

14. Mélange de dérivés de triazine selon l'une des revendications précédentes, comprenant au moins une résine d'urée, une résine de mélamine - urée, une résine de mélamine, une résine de mélamine - phénol, une résine de phénol, un polyester, un polyacrylate, un polyuréthane, un époxyde, un polyéther et/ou un mélange de ceux-ci.

15. Utilisation des dérivés de triazine selon l'une des revendications 1 à 13 ou du mélange selon la revendication 14 comme agents de réticulation et/ou comme liants dans des matières de revêtement, des vernis et des peintures, comme liants pour des matériaux composites et matières à mouler, dans des mousses, dans des fibres et conjointement avec d'autres substances dans des équipements anti-incendies, comme agents de réticulation et/ou co-réactifs lors de la fabrication d'un stratifié pour améliorer les propriétés de surface du stratifié.

16. Procédé de fabrication de dérivés de triazine selon l'une des revendications 1 à 13,
**caractérisé par**
A) une réaction d'un dérivé de triazine de formule générale (IX) 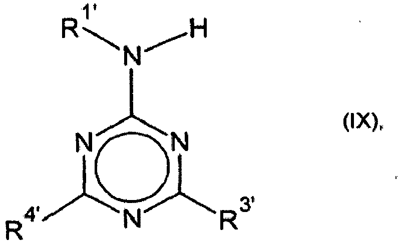 dans laquelle
- R^{3'} représente Q¹ ou un radical lié à l'atome d'azote central sur le cycle triazine de structure de formule (IX), ayant la formule R^{5'}-N-R^{6'}, dans lequel
-- Q¹ représente un alkyle en C₁-C₅₀ linéaire ou ramifié ou un substituant cyclique sous la forme d'un cycloalkyle en C₅-C₂₀, d'un aryle en C₅-C₂₀, d'un aryle en C₅-C₂₀ substitué par un alkyle en C₁-C₂₀, d'un aryle en C₅-C₂₀ substitué par un alcényle en C₂-C₂₀ ou d'un imide d'acides carboxyliques cycliques saturés ou insaturés, où l'alkyle en C₁-C₅₀ ou le substituant cyclique peut être interrompu par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-,-OC(O)NH- et/ou -OC(O)O-,
- R^{4'} représente Q¹ ou un radical lié à l'atome d'azote sur le cycle triazine de structure de formule (IX), ayant la formule R^{7'}-N-R^{8'},
- R^{1'}, R^{5'} et R^{7'} représentent, indépendamment les uns des autres, Q², dans lequel
-- Q² représente respectivement un alkyle en C₁-C₅₀ linéaire ou ramifié, un cycloalkyle en C₅-C₂₀, un aryle en C₅-C₂₀, un aryle en C₅-C₂₀ substitué par un alkyle en C₁-C₂₀, un alcényle en C₂-C₂₀ ou un aryle en C₅-C₂₀ substitué par un alcényle en C₂-C₂₀, qui peut être interrompu à chaque fois par un ou plusieurs atomes d'oxygène, atomes de soufre, atomes d'azote substitués et/ou non substitués, et/ou par un ou plusieurs groupes du type -C(O)O-, -OC(O)-, -C(O)-, -NHC(O)O-, -OC(O)NH- et/ou -OC(O)O-,
- R^{6'} représente H, Q², -CO-A-R⁶¹ ou -CO-A-R⁸¹,
- R^{8'} représente H, Q², -CO-A-R⁶¹ ou -CO-A-R⁸¹,
- A représente -O-, -NH- ou -NR⁶²- ou NR⁸²-,
- R⁶¹, R⁶², R⁸¹ et R⁸² représentent, indépendamment les uns des autres, Q², un radical d'un alcool substitué ou non substitué, un radical d'un alcool polyvalent substitué ou non substitué, un radical d'une amine, un radical d'un aminoalcool, un radical d'une diamine ou un radical de formule générale (II) 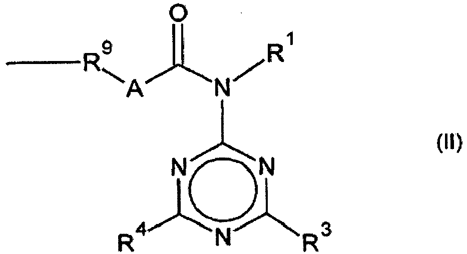
- dans laquelle R⁹ représente Q²,
avec du phosgène, et
B) une réaction ultérieure du chlorure de carbamoyle du dérivé de triazine, formé à l'étape A), avec un alcool substitué ou non substitué de formule générale R²-OH, un alcool polyvalent substitué ou non substitué de formule générale R²-(OH)ₙ avec n ≥ 2, une amine de formule générale R²-NH₂ ou R²-NH-R²⁰, un aminoalcool de formule générale OH-R²-NH₂ ou R²-NH-R²⁰-OH, une diamine de formule générale R²-(NH₂)₂ ou un radical de formule générale (II),
- dans lesquelles R² et R²⁰ représentent, indépendamment les uns des autres, Q², un radical d'un alcool substitué ou non substitué, un radical d'un alcool polyvalent substitué ou non substitué, un radical d'une amine, un radical d'un aminoalcool, un radical d'une diamine ou un radical de formule générale (II).
